# EUROPEAN PATENT APPLICATION

(11) **EP 3 772 356 A1**
(43) Date of publication of application: **10.02.2021**
(21) Application number: 19190593.4
(22) Date of filing: 07.08.2019
(51) Int. Cl.: A61K 47/64, A61K 47/69, A61K 9/51, A61K 49/00, A61K 41/00, A61P 35/00

(54) **TARGETED NANOPARTICLES OF WELL-DEFINED AND REPRODUCIBLE SIZES**

(71) Applicant: Stams Diagnostics GmbH, 6433 Oetz (AT)
(72) Inventor: Debbage, Paul, 6433 Oetz (AT); Thurner, Gudrun, 6433 Oetz (AT)
(74) Representative: Grünecker Patent- und Rechtsanwälte PartG mbB

(57) **Abstract**

The invention relates to a nanoparticle preparation comprising nanoparticles of well-defined and reproducible sizes, that are substantially free of aggregate formation.

## Description

### I. Field of the invention

The invention relates to nanoparticle preparations for molecular imaging or drug delivery, comprising organic nanoparticles having a diameter of less than about 100 nm, wherein the nanoparticles have a narrow size distribution, e.g. wherein 90% of the organic nanoparticles have a diameter within 25 nm of the average diameter of the organic nanoparticles comprised in the nanoparticle preparation, wherein the nanoparticle preparation is substantially monodisperse, and wherein the surface charge of the organic nanoparticles at pH 7 is about zero, and methods of preparing the same. The invention further relates to nanoparticle preparations comprising a targeting agent, a signaling agent, an active agent or any combination thereof. The invention further relates to compositions comprising the nanoparticle preparation of the invention. The invention further relates to a nanoparticle preparation or the composition for use in therapy or diagnostics, such a theranostics. The invention further relates to a nanoparticle preparation or composition for use in a method of treating cancer. The invention further relates to a nanoparticle preparation or composition for use in diagnosing and/or detecting epithelial lesions, in particular carcinoma. The invention further relates to an imaging method using the nanoparticle preparation or composition of the invention. The invention further relates to the use of the nanoparticle preparation or composition of the invention.

### II. Background of the invention

Carcinoma, i.e. cancer originating from an epithelium, is one of the leading causes of death in the world, with around 400,000 deaths annually alone in the USA, and about 800,000 deaths annually world-wide.

In view of the high prevalence of carcinoma, and the poor outcome associated with a later diagnosis, early detection of cancer is needed and would significantly increase the chances for successful treatment. Disease diagnoses delayed by failure to detect early lesions result in increased morbidity and mortality, with associated socio-economic costs.

Pre-malignant lesions of the epithelium, i.e. regions of abnormal or irregular epithelia, are difficult to detect, as often the patient is under no pain. These pre-malignant lesions therefore go often unnoticed for prolonged periods of time.

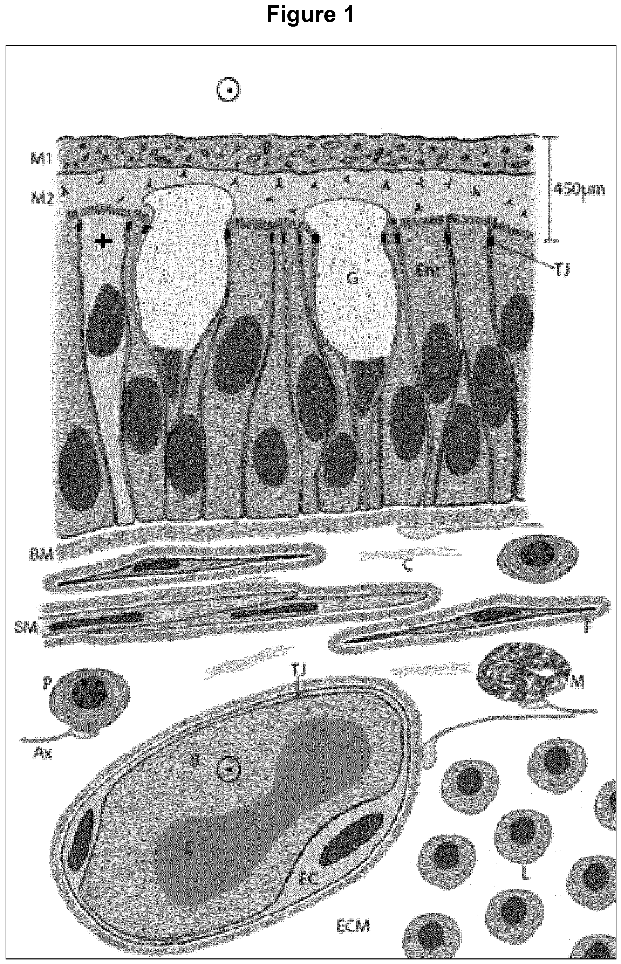

Using targeted nanoparticles for detecting or treating cancer has been attempted in the past without much success. One of the obstacles is that the nanoparticles are very large and unstable, and after administration to the patient accumulate to large extent in the liver. Further, the administration by intravenous injection, which is the most common route for administration of nanoparticles, is further hindered by the limited biodistribution. The limited biodistribution, which is partly due to numerous barriers in the body (Figure 1), results in concentration of nanoparticles at unwanted sites, e.g. liver, whereas the remaining few circulating nanoparticles do not come into contact with the target cells . One of the impediments in nanoparticle-based tumor targeting is the inability to limit the undesired trafficking of nanoparticles to the liver and other organs.

The blood space as a body cavity: This epithelium (ie the endothelium) can be accessed apically from the blood. The endothelium is the site of inflammatory lesions that underlie atherosclerosis. This disease is of acute clinical interest and is much researched. Its lesions are susceptible to targeted imaging from within the bloodstream.

The joint cavity between bones is a further site to which the nanoparticle can be applied. The flat cells lining joint cavities are not epithelial cells, but the same principle applies that the nanoparticles are introduced directly to their site of action without being filtered/cleared in the blood.

Manufacturing methods of the art for preparing protein nanoparticles are generally based on emulsification, desolvation, coacervation and different combinations thereof. Emulsification bears problems of stability and dispersity, desolvation bears problems of using organic solvents and protein precipitation disturbing the native protein structure, and coacervation normally is used in combination with desolvation using organic solvents.

The term coacervation was first introduced by H. G. Bungenberg de Jong and H. R. Kruyt in 1930 [Thomasin et al., 1998], who described the separation of a liquid colloidal phase, such as an aqueous gelatin solution, into a colloid-rich layer, the coacervate, and a colloid-poor layer. Following this terminology and taking into account the different chemical and physical mechanisms of phase separation, this concept was refined by division into simple and complex coacervation. Simple coacervation salts out a polymer using electrolytes, whereas complex coacervation desolves a polymer by use of different organic solvents, e.g. CH₂Cl₂ or ethyl acetate, which act as non-solvents to the polymer. In both cases the polymer precipitate shows a rather broad size distribution, and in addition, if the polymer used is a protein, then its natural conformation may be altered. Also, the biocompatibility of such coacervated particles may be reduced because trace amounts of solvents might remain [Thomasin et al., 1998]. In contrast to these conventional types of coacervation the method described in this thesis does not lead to any precipitation of the protein, nor does it leave remnant traces of toxic organic materials. Even though some of the critical synthesis parameters have been elucidated, i.e. the pH value, the protein and the salt concentration, the temperature, the stirring speed and the concentration of the linking molecule, it is not yet entirely clear why this method works. It is obvious that a thermodynamically driven reaction between the interfaces of adjacent HSA molecules, caused by the presence of salt and heat, precedes the formation of these particles.

For screening of populations and for monitoring of therapies it is generally sufficient to detect that a lesion is present or absent, since the indication that a lesion may be present serves as clinical indication for further testing, and a simple count of lesions often suffices for monitoring therapy.

Lastly, the known nanoparticles are not ideal for targeted nanoparticle-based methods, because the known methods result in nanoparticles that are very heterogeneous in size and further carry an electrical charge. In fact, the general teaching in the art considers the negative charge of nanoparticles to be essential.

There is thus a need for a method that allows early detection of epithelial lesions, including pre-cancerous lesions, and cancerous lesions of varying stages. Further, there is a need for an imaging or detecting method of epithelial lesions, wherein the nanoparticle does not accumulate in the body at undesirable sites, enables detecting of the signal even from outside the body, is stable and easy to manufacture. It is an object of the invention to address such needs.

### III. Object of the invention

Specifically, one object of the invention is to provide a composition, comprising nanoparticle preparation, that can be advantageously used in imaging methods, detection methods, diagnostic methods and therapeutic methods. Such a nanoparticle preparation may be directly applied to the body cavity of a subject, thereby minimizing the distance the nanoparticles comprised in the nanoparticle preparation has to travel to the target site, and more importantly avoiding the numerous clearance mechanisms and barriers of the body which prevent efficient localization of the nanoparticle to the target site. Yet another object of the invention is to provide a nanoparticle preparation that comprises nanoparticles having a global surface charge of about zero, thereby preventing unspecific adhesion to positively charged materials and repulsion from negatively charged materials, as occurs with negatively-charged nanoparticles. A further object of the invention is to provide a nanoparticle preparation that is substantially aggregate-free. Yet another object of the invention is to provide a nanoparticle preparation that is monodisperse, i.e. has a narrow size distribution of the nanoparticles which can be reliably reproduced from batch to batch of the preparations . Yet another object of the invention is to provide a nanoparticle preparation, wherein the nanoparticles comprised therein are small enough for uptake by the target cell by receptor-mediated endocytosis, for example 30 nm diameter.

The compositions, for example nanoparticle preparations and compositions, as well as methods and uses involving such compositions described below, satisfy the above aims.

### IV. Summary of the invention

Presented herein are preparations, compositions, methods and uses for targeted delivery of imaging agents and/or therapeutic agent to sites of disease in a subject by attaching the agent to an organic nanoparticle.

The organic nanoparticle provided herein enables the detection of epithelial lesions in a subject, wherein the nanoparticle preparation is monodisperse, and further wherein the small size of the nanoparticle and its surface charge of about zero, enable the uptake of the nanoparticles into the cells, preferably by receptor-mediated endocytosis.

In one aspect, the organic nanoparticle provided herein is stable and substantially aggregate free.

The nanoparticle preparation and compositions provided herein may be applied directly into the body cavity, e.g. the colorectal cavity, thereby overcoming the limitations associated with tissue barriers and dilution in the blood system, enhancing the rate of detection, and reducing undesirable side effects.

For example, following administration of the nanoparticle preparation or composition to a subject, the targeting group will specifically target the nanoparticle to the target site, such as a precancerous epithelial lesion. Due to the small size of the nanoparticle, the nanoparticle will be endocytosed into the target cells. The active agent and/or signaling agent attached to the nanoparticle, will also be delivered to the lesion site. The nanoparticle preparation will thus specifically label the site of the precancerous epithelial lesion. Depending on the nature of the signaling agent, the lesion may be monitored from inside (e.g. during endoscopy) or outside (e.g. by off-peak photoluminescent imaging or Magnetic resonance imaging) the subject's body.

The earliest pre-cancerous changes of a tissue are not visible, and occur when no symptoms are yet present. While biopsies provide valuable information, it is not feasible to take tissue biopsies when no symptoms are present, from a vast area of tissues.

The present invention allows the fast and quick detection of early changes of the epithelium, which would not be detectable using routine optic-based exams. The early detection of hyperplastic tissue, which may result in carcinoma, is a valuable tool to diagnose a disease, monitor disease and/or therapy progression, deliver therapeutic agents or any combination of the same.

Thus, in certain embodiments, methods and compositions are provided herein that use organic nanoparticles to deliver therapeutic agents to sites of disease. In certain embodiments, methods and compositions are provided herein that use organic nanoparticles to deliver signaling or imaging agents to sites of disease, such as hyperplastic epithelia or cancer.

The organic nanoparticle is preferably non-covalently associated with an active agent. The organic nanoparticle, preferably a polypeptide nanoparticle, most preferably a HSA nanoparticle, is able to retain active agents, such as drugs, without chemical modification of the agent.

The nanoparticle maintains its association with the targeting group, signaling agent or active agent through covalent or non-covalent interactions. The active agent and/or signaling agent may be released at the site of the target cell, either after specifically binding to the target cell, or after endocytosis into the target cell.

In one aspect, the invention is directed to a nanoparticle preparation for molecular imaging or drug delivery, comprising organic nanoparticles having a diameter of less than about 100 nm, preferably about 10 nm - about 80 nm, more preferably about 20 nm - about 60 nm, even more preferably about 20 nm - about 50 nm, most preferably about 30 nm, wherein at least 90% of the organic nanoparticles have a diameter within 25 nm of the average diameter of the organic nanoparticles comprised in the nanoparticle preparation, wherein the nanoparticle preparation is substantially monodisperse, and wherein the surface charge of the nanoparticles at pH 7 is about zero. In some preferred embodiments, the at least 90% of the organic nanoparticles have a diameter within 20 nm, within 15 nm, within 10 nm, or within 5 nm, of the average diameter of the organic nanoparticles comprised in the nanoparticle preparation. In a more preferred embodiment, the invention provides a nanoparticle preparation for molecular imaging or drug delivery, comprising organic nanoparticles having a diameter of about 20 nm - about 50 nm, preferably about 30 nm, wherein at least 90% of the organic nanoparticles have a diameter within 5 nm of the average diameter of the organic nanoparticles comprised in the nanoparticle preparation, wherein the nanoparticle preparation is substantially monodisperse, and wherein the surface charge of the nanoparticles at pH 7 is about zero.

In one aspect, the nanoparticle preparation is substantially aggregate free. In some embodiments, less than 10 %, less than 9 %, less than 8 %, less than 7 %, less than 6 %, less than 5 %, less than 4 %, less than 3 %, less than 2 %, or less than 1 % of the nanoparticles comprised in the nanoparticle preparation of the invention form aggregates.

Another aspect of the invention relates to a composition comprising the nanoparticle preparation according the invention, wherein said composition is in form of a dispersion, a liquid, suspension, a powder, encapsulation, aerosol or gel.

In another aspect the invention relates to a nanoparticle preparation or composition according to the invention for use in therapy or diagnostics.

In another aspect the invention relates to a nanoparticle preparation according to the invention for use in a method of diagnosing a disease caused by an epithelial lesion, preferably a carcinoma.

In another aspect the invention relates to a nanoparticle preparation according to the invention for use in a method of treating cancer, wherein said cancer is preferably carcinoma, more preferably, wherein said carcinoma is colorectal carcinoma, prostate cancer, hepatocellular carcinoma, breast cancer, lung cancer, small cell lung cancer, non-small cell lung cancer, or lung adenocarcinoma.

In another aspect, the invention relates to a method of making a nanoparticle preparation, said method preferably comprising the steps of
- Providing an aqueous solution of an organic molecule in its native conformation at pH 4 to pH 8, and at a concentration of about 1 mg/ml to about 50 mg/ml, wherein the organic molecule is preferably HSA;
- Adding an inorganic salt, preferably an alkali salt such as sodium chloride, at a concentration of 0.5 to 2 M under continuous stirring and at a temperature of about 40 to about 65 °C for a time period to allow coacervate formation; and
- Adding a cross-linking agent, such as glutaraldehyde, 1,5-dichloropentane or glutarylchloride, at a final concentration of about 0.05 %(w/v) to 0.2 % (w/v), preferably wherein the final concentration is 0.1 % (w/v), .

In another aspect the invention relates to a method of detecting an epithelial target site in an organism, wherein the target site is preferably associated typically with a lesion, said method comprising
- administration of the nanoparticle preparation or composition according to the invention to a body cavity; and
- detecting a signal emitted by the nanoparticle located at the target site,
wherein the lesion is preferably selected from the group consisting of an inflammatory lesion, a dysplastic epithelium, an early-stage carcinoma, a metastatic malignancy, an infectious lesion, wherein the lesion preferably comprises a viral antigen, bacterial antigen, fungal antigen, protozoan antigen, or worm antigen.

In another aspect the invention relates to a method for imaging an epithelial lesion, said method comprising
- administration of the nanoparticle preparation or composition according to the invention to a body cavity; and
- detecting a signal emitted by the nanoparticle located at the target site;
wherein the body cavity is preferably selected from the group consisting of the colon, oral cavity, ear canal, prostate, lung, oral cavity, ear-nose-throat canals, oesophagus, stomach, duodenum, jejunum, ileum, colon, sigmoid colon, rectum, anus, trachea, bronchioli and lung alveoli, vagina, cervix uteri, uterus, urethra, and urinary bladder,
wherein the preferred mode of administration of the nanoparticle preparation to the body cavity is by any suitable route, including orally, intravenously, intramuscularly, intraperitoneally, subcutaneously, by inhalation, intra-arterially, intramedullary, intrathecally, subcutaneously, transdermally, interdermally, intradermally, rectally, vaginally, intraperitoneally, intragastrically, topically, mucosally, intranasally, buccally, enterally, vitreally, sublingually, by intratracheal instillation, bronchial instillation, and/or inhalation; as an oral spray, nasal spray, and/or aerosol, and/or through a portal vein catheter. In a preferred embodiment, the nanoparticles are administered by endoscopy or non-invasive routes, e.g. orally or by inhalation. In some embodiments, the dosage form is a powder, ointment, cream, gel, lotion, drop, lozenge, or spray.

In another aspect the invention relates to a use of a nanoparticle preparation or composition according to the invention for imaging or detecting a lesion.

In another aspect the invention relates to the nanoparticle preparation or composition obtainable by the methods provided herein.

### V. Description of the Figures

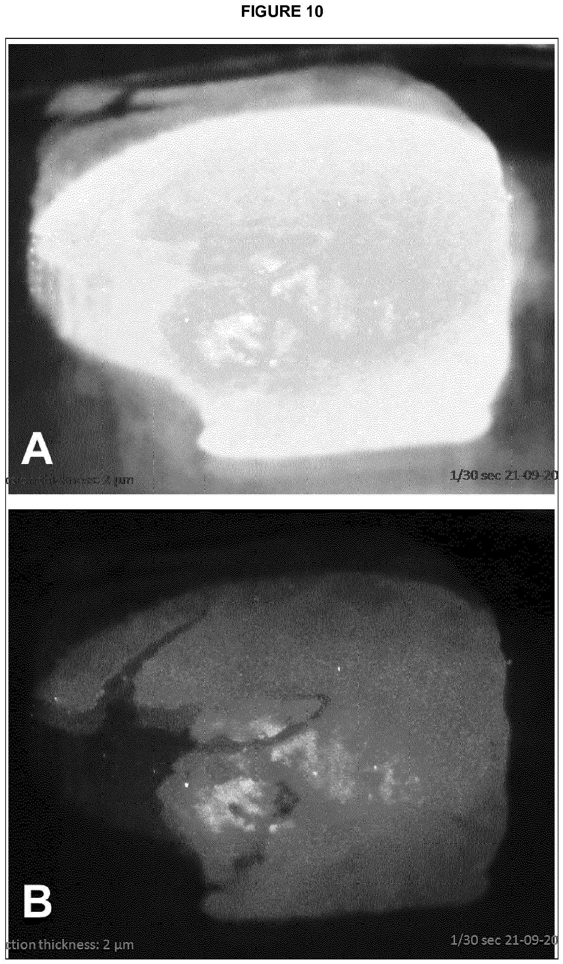

**Figure 1****:**
   This sketch shows the numerous barriers which tissue structure places between a nanoparticle (circled) in the blood stream and a target molecule located at the apical side of an epithelial cell (marked with a cross). This Figure is taken from *Debbage & Thurner (2012).*
**Figure 2****:**
   **A:** Routine examination of the nanoparticles of the invention by negative contrast electron microscopy revealed 1) the homogeneity of their sizes, 2) their size of about 30 nm and 3) their intrinsic absence of aggregates (filtration was not necessary).
   **B:** Individual nanoparticles prepared according to the inventive method are densely packed and spheroidal and have a diameter of about 30 nm.
**Figure 3****:**
   By varying the synthesis conditions the nanoparticles disclosed here can be produced in a range of sizes.
   **A:** Photon Correlation Spectroscopic (PCS) measurements of the same batches of nanoparticles already viewed by Electron Microscopy confirmed 1) the sizes of the nanoparticles, 2) the homogenous sizes of the nanoparticle populations and 3) the absence of aggregates. The batch shown here was synthesized according to a protocol designed to produce nanoparticles of 30 nm diameter.
   **B:** The same comments apply as for **3A,** but this batch was synthesised according to a protocol designed to produce nanoparticles of 70 nm diameter.
**Figure 4****:**
   **A to F:** show 6 batches of the inventive nanoparticles synthesized according to a protocol designed to produce nanoparticles of 30 nm diameter. The batches formed a consecutive series and were prepared at intervals of several weeks between the consecutive batches. This Figure shows the reliability and reproducibility of the protocol. The absence of aggregate formation is highly reproducible.
**Figure 5****:**
   **A:** The global surface charge of the cleaned and stabilized albumin molecules used as starting material to prepare the nanoparticles was found by PCS based measurement of the Zeta Potential to be about -5 mV. *Peters* (1996) describes a preponderance of negatively charged groups at the surface of the albumin molecule at pH 7.
   **B:** The same measurement carried out on the nanoparticles prepared according to the invention showed their global surface charge to be zero.
**Figure 6****:**
   This Figure documents **transendothelial transcytosis** of 30 nm diameter albumin nanoparticles in **A**) rabbit kidney, **B**) human term placenta and **C**) rat capillary after HSA nanoparticles (see white, grey and black arrows) were injected intravenously. **A)** shows the nanoparticles (arrows) crossing the kidney's ultrafilter; **B)** shows the nanoparticles crossing a capillary endothelial cell into the subvascular connective tissue (arrows). **C)** shows endocytosis (arrow) of the nanoparticles from the vascular lumen (L), transcytosis of the nanoparticles across the endothelial cell cytoplasm (two of them are indicated in white rings) and exocytosis (arrow) into the subendothelial basement membrane (BM) at bottom right of the Figure.
**Figure 7****:**
   Sketch showing the imaging procedure which renders the fluorochrome-bearing targeted nanoparticles visible by fluorescence. After topical application of the nanoparticles (left side of Figure) the specifically bound nanoparticles internalise into the epithelial cells during three to five minutes and then fluoresce brightly from the cytoplasm of the target cells.
**Figure 8****:**
   Thin Layer Chromatography showed that the PP IX was anchored to the stabilized HSA: the fluorescence remained at the starting point of the TLC together with the protein (Dot 2), whereas unbound PP IX moved with the advancing edge of the solvent (Dot 1).
**Figure 9****:**
   **A:** This fluorescence image of nanoparticles prepared according to the inventive method shows that the PP IX remained fluorescent and stably anchored to the nanoparticles even under the harsh denaturing conditions of Sodium Dodecyl Sulphate Polyacrylamide Gel Electrophoresis.
   **B:** Coomassie Blue staining after Sodium Dodecyl Sulphate polyacrylamide gel electrophoresis showed that the invented nanoparticles were stable under the harsh conditions of this method (denaturing effect of SDS and heat), with only minimal break up into smaller pieces. The nanoparticles, being too large to enter the gel, are visible as black masses at the very start of the gel's insertion wells.
**Figure 10****:**
   Fluorescence imaging of tumour cells in a human colon carcinoma by PP IX-bearing EpCAM-targeted HSA nanoparticles binding specifically to them.
   In **A**) the (blue) excitation light is reflected from the tumour tissue.
   In **B**) the (pink) fluorescence emitted from the nanoparticles indicates the location of the EpCAM expressing tumour cells
**Figure 11****:**
   **A**) The same tumour tissues already shown in **Figure 10** were processed to show the distribution of HSA in the tissue; the procedure reveals the nanoparticles because they contain HSA and not because they contain fluorescent PP IX; in this image they appear black (white arrow). The distribution matches that shown by PP IX fluorescence in **Figure 10****.** The point marked with a black arrow is shown in **B**) at higher resolution and the HSA nanoparticles are seen to be located on the tumour cells (arrow).
**Figure 12****:**
   This Figure shows that the targeting specificity of the PP IX bearing HSA nanoparticles depends **only** on the targeting group attached to them. In this Figure, the targeting group on the nanoparticles was an antibody directed against GFAP. The nanoparticles bind specifically to the astrocytes in this brain tissue. The images **A-D** shows a series of control experiments performed on tissues from *Callithrix* brain, and the images **E-H** show the same controls carried out on tissues from a human brain tumour (a glioma).

### VI. Detailed description of the invention

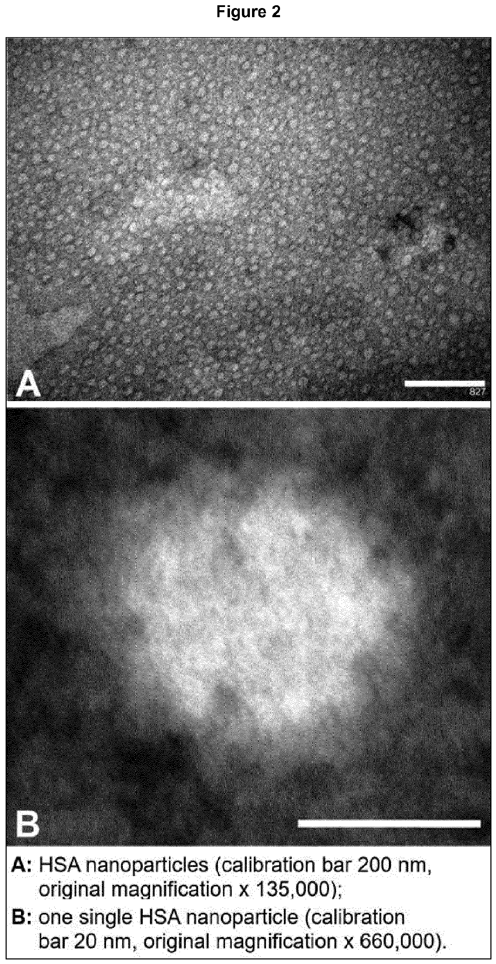

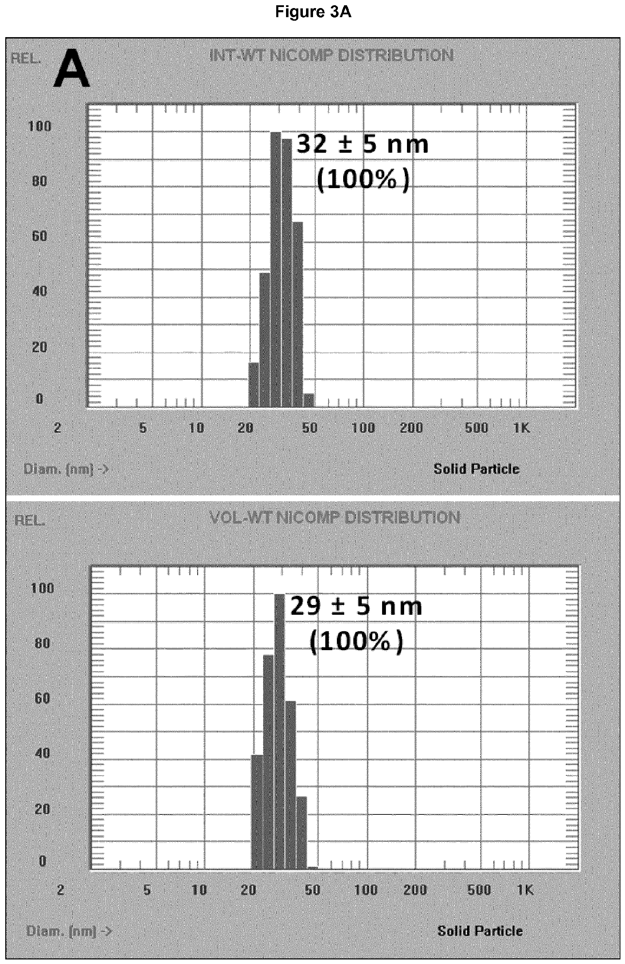

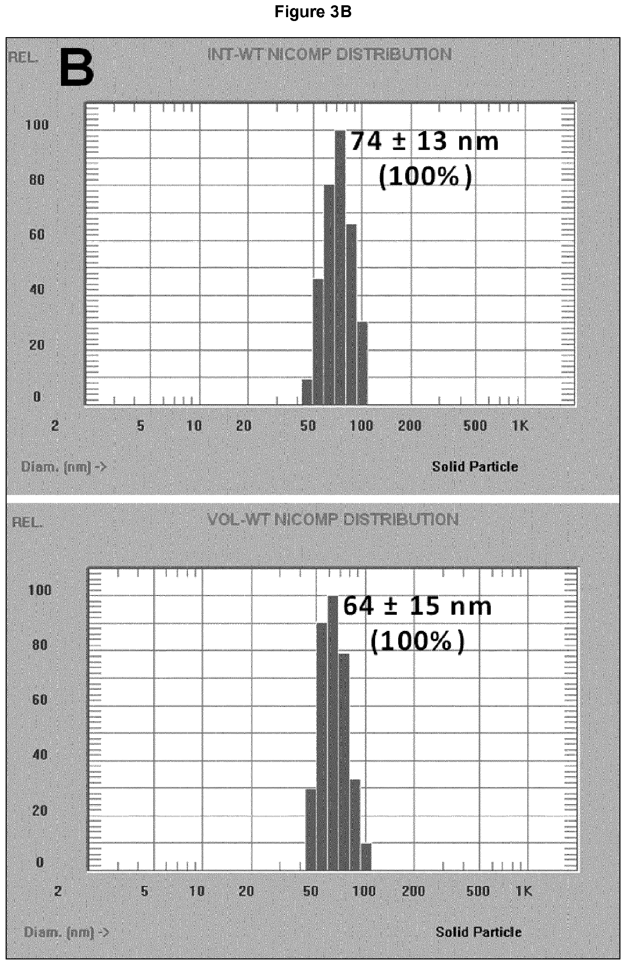

The present invention provides methods, uses, compositions, and kits for imaging, diagnosing, detecting, or treating of an epithelial lesion, preferably cancer using organic nanoparticles. The nanoparticle preparation provided herewith is monodisperse, stable and further characterized by comprising nanoparticles of small size, i.e. having a diameter of less than 100 nm **(****Figures 2-4****).**

The present invention further provides methods, compositions, uses and kits for use as a medicament. The present invention further provides methods, compositions, uses and kits for use in a method of treating a disease, preferably wherein said disease is a carcinoma. The present invention further provides methods, compositions, uses and kits for use in a method of diagnosing a disease, preferably wherein said disease is a carcinoma.

Any of the nanoparticle preparations or compositions provided herewith can be used to image, detect, monitor, treat, stabilize, prevent, and/or delay cancer.

### A. Definitions and terminology

Unless defined otherwise, all technical and scientific terms used herein have the same meaning as commonly understood by one of ordinary skill in the art.

Unless defined otherwise, any feature within any aspect or embodiment of the invention may be combined with any feature within any other aspect or embodiment of the invention, and the skilled person understands such combination as being encompassed in the original disclosure of the present application. This applies in particular to all embodiments described within the section relating to compositions per se, in respect of other aspects, e.g. methods and uses, of those compositions. This also applies in particular, but not exclusively, to endpoints of ranges disclosed herein. For instance, if a given substance is disclosed as existing in a composition in a concentration range of X-Y% or A-B%, the present application is to be understood as explicitly disclosing not only the ranges X-Y% and A-B%, but also the ranges X-B%, A-Y% and, in as far as numerically possible, Y-A% and B-X%. Each of these ranges, and range combinations, are contemplated, and are to be understood as being directly and unambiguously disclosed in the present application.

As used herein, the term "average diameter", when applied to nanoparticles, refers to the average diameter of the nanoparticles in a sample, such as a nanoparticle preparation, wherein the average diameter is an average hydrodynamic diameter. The average diameter is measured by dynamic light scattering also known as Photon Correlation Spectroscopy (PCS).

As used herein, the term "administration" of an agent to a subject includes any route of introducing or delivering the agent to a subject to perform its intended function. Administration can be carried out by any suitable route, including orally, , intraperitoneally, subcutaneously, by inhalation, subcutaneously, transdermally, interdermally, intradermally, rectally, vaginally, intragastrically, topically, mucosally, intranasally, buccally, enterally, vitreally, sublingually; by intratracheal instillation, bronchial instillation, and/or inhalation; as an oral spray, nasal spray, and/or aerosol. In a preferred embodiment, the nanoparticles are administered by endoscopy or non-invasive routes, e.g. orally or by inhalation. In some embodiments, the preferred dosage form is a powder, ointment, cream, gel, lotion, drop, lozenge, or spray.

Unless stated otherwise, percentages indicating the extent to which a particular ingredient is comprised in a mixture or composition refer to weight/weight (w/w) percentages. Similarly, unless stated otherwise, ratios specified herein are weight ratios.

As used herein, the term "monodisperse" refers to a composition that remains in solution phase, e.g. as a dispersion, and does not sediment or form aggregates after an extended period of time, e.g., 1 hour,2 hours, 1 day, 3 days, 5 days, 1 week, 1 month, 3 months, 6 months, 1 year, 2 years or more).

As used herein, "coacervate" refers to a colloidal solution of homogenous protein nanoparticles which we consider to consist of soft matter. In one embodiment, a coacervate is formed during the preparation method of the invention.

As used herein, "room temperature" denotes a typical ambient indoor temperature of about 25°C. "Ambient temperature" and "room temperature" are used interchangeably hereinafter.

As used herein, the term "active agent" denotes a substance that has a medically relevant activity. In some embodiments, the active agent is a chemotherapy drug, antimetabolite agents (including nucleoside analogs), a cytotoxic agent (e.g. a toxin), alkylating agents, tyrosine kinase inhibitors, anthracycline antibiotics, vinca alkloids, proteasome inhibitors, taxanes, modulators of HER2/neu (such as inhibitors of HER2/neu for example Herceptin®), modulators of EGFR (such as inhibitors of EGFR for example Erbitux®), modulators of VEGFR, farnosyltransferase inhibitors, and topoisomerase inhibitors. In some embodiments, the chemotherapeutic is not a taxane(i.e., the compound is a chemotherapeutic agent other than a taxane).The active agent described herein can be the agent itself, or a pharmaceutically acceptable salt thereof, pharmaceutically acceptable ester thereof, as well as stereoisomer, enantiomer, racemic mixture, and the like.

The term "hydrodynamic diameter" has its plain ordinary meaning within Chemistry and refers to the apparent diameter of a hypothetical hard sphere that diffuses through a medium at the same speed as the molecule under observation (e.g. as measured by dynamic light scattering or Photon Correlation Spectroscopy (PCS)).

As used herein, the term "cross-linking agent" refers to a compound which stabilizes the coacervate, resulting in a stable nanoparticle. Cross-linking agents in the sense of the invention enable linkage formation between the organic molecules of the nanoparticle. In one embodiment, the cross-linking agent is glutaraldehyde, 1,5-dichloropentane or glutarylchloride. In a preferred embodiment, the organic nanoparticle is a HSA nanoparticle cross-linked using glutaraldehyde. Glutaraldehyde makes a linker between primary amino groups of HSA molecules and can link two HSA molecules together. In some embodiments, the cross-linking agent is non-toxic. In some embodiments, the cross-linking agent comprises 8, 7, 6, 5, 4, or 3 carbon atoms.

As used herein, the term "dispersion" has its plain ordinary meaning within the field of Chemistry and refers to a system containing particles dispersed in a continuous phase of a different composition (e.g. nanoparticles or nanoparticles dispersed in a liquid phase). In some embodiments, a dispersion may be a suspension, wherein a suspension has its plain ordinary meaning within Chemistry and refers to a dispersion of solid particles in a continuous liquid phase, wherein the solid particles are large enough for sedimentation. In some embodiments, a dispersion may be a colloid, wherein a colloid has its plain ordinary meaning as used within Chemistry. In some embodiments, a dispersion comprises nanoparticles dispersed in a continuous liquid phase. In some embodiments the dispersed particles are protein nanoparticles. In some embodiments, a dispersion liquid is a liquid solution in which protein nanoparticles are dispersed. In some embodiments, a dispersion liquid is a non-aqueous liquid in which protein nanoparticles are dispersed. In some embodiments, a dispersion liquid is an aqueous liquid in which protein nanoparticles are dispersed.

As used herein, the term "non-glycosylated protein" refers to a protein lacking carbohydrate or sugar moieties covalently attached to said protein. Glycosylation is critical for a wide range of biological processes, including cell attachment to the extracellular matrix and protein-ligand interactions in the cell. Glycosylation comprises various glycosidic linkages, including N-, O- and C-linked glycosylation, glypiation (GPI anchor attachment), and phosphoglycosylation. Glycoproteins can be detected, purified and analyzed by different strategies, including glycan staining and visualization, glycan crosslinking to agarose or magnetic resin for labeling or purification, or proteomic analysis by mass spectrometry, respectively. In some embodiments, the nanoparticle of the invention comprises a non-glycosylated protein. In some embodiments, the non-glycosylated protein is a de-glycosylated protein, wherein the carbohydrate or sugar moieties attached to glycosylated proteins are removed by de-glycosylation. In a preferred embodiment, the non-glycosylated protein is human serum albumin (HSA), transferrin, apo-transferrin, streptavidin, granulocyte colony stimulating factor, or insulin. In a more preferred embodiment, the non-glycosylated protein is human serum albumin (HSA).

In some embodiments, the continuous phase of a different composition comprises protein in solution. In some embodiments, the protein in solution is less than about 50%, 40%, 30%, 20%,10%, 9%, 8%, 7%, 6%, 5%, 4%, 3%, 2%, 1%, 0.5%, 0.4%, 0.3%, 0.2%, 0.1%, of the total protein in the dispersion (i.e. particles and continuous phase combined).

As used herein, the term "aggregate" or "nanoparticle aggregate" refers to a plurality of nanoparticles, preferably polypeptide nanoparticles, in a liquid medium, which form aggregates and are unstable. An aggregated nanoparticle may also be termed an "unstable" nanoparticle.

As used herein, the terms "stable nanoparticle", "stable nanoparticle preparation", and "stable nanoparticle solution" refer to a nanoparticle, nanoparticle preparation or nanoparticle solution, respectively, which do not form substantial amounts of aggregates at pH 7, wherein the substantial amount refers to an amount of less than 10 %, less than 8%, less than 5, less than 3%, or less than 1% of the totality of the nanoparticles present in a nanoparticle preparation or nanoparticle solution. In one aspect, the nanoparticle preparation of the invention comprises stable nanoparticles. In one embodiment, the stable nanoparticles, stable nanoparticle preparation or stable nanoparticle solution have a shelf-life of at least 1 month, 3, months, 6 months, 9 months, 1 year, 2 years, or more.

A "signaling agent" is a compound detectable by spectroscopic, photochemical, biochemical, immunochemical, chemical, or other physical means. For example, useful labels include 32P, fluorescent dyes, electron-dense reagents, enzymes {e.g., as commonly used in an ELISA), biotin, digoxigenin, or haptens and proteins or other entities which can be made detectable, e.g., by incorporating a radiolabel into a peptide or antibody specifically reactive with a target peptide. Any method known in the art for conjugating a targeting group, such as an antibody, to the label may be employed. As used herein, the term "plurality" refers to more than one.

The terms "patient", "subject", "individual", and the like are used interchangeably herein, and refer to an animal, typically a mammal. Subjects that may be treated using the method of the invention include, but are not limited to humans, cows, horses, pigs, dogs, cats, sheep goats, rabbits, rats, mice, birds or chickens. In a preferred embodiment, the patient, subject, or individual is a mammal. In a particularly preferred embodiment, the patient, subject or individual is a human.

The phrase "targeted binding", refers to the selective and specific binding of the targeting group to the target site. As an example, in case of a antibody being the targeting group, the targeted binding refers to the specific binding of said antibody to its antigen (referred to target suite hereinafter) on the target cell.

As used herein, the term "targeting group" refers to a moiety that has specific binding capability to a target site on a tissue of a subject. In one embodiment, the nanoparticle of the invention comprises one targeting group on its surface. In another embodiment, the nanoparticle of the invention comprises a plurality of targeting groups on its surface. In a preferred embodiment, the targeting group is an antibody, or a fragment thereof. In a preferred embodiment, the nanoparticle preparation comprises a plurality of nanoparticles, wherein the nanoparticles comprise targeting groups directed at different target sites.

As used herein, the term "linker" refers to a chain of residues bearing a specific reactive group at each end, and which connect or link a targeting group, a signaling agent, or active agent to the nanoparticle. In some embodiments, the linker possesses a minor lateral flexibility. In some embodiments, the linker is enzymatically cleavable from said nanoparticle. In some embodiments, the linker has a neutral charge. The linker is preferably an alkyl chain between C1 and C20, wherein the alkyl chain length will be longer for a larger targeting group, a signaling agent, or active agent. In preferred embodiments, the linker is an alkyl chain, that can freely rotate, i.e. has no double and triple bonds. I some embodiments, the linker comprises at least one mono- or bivalent functional group at each end of the linker in order to attach the targeting group, signaling agent or active agent to the nanoparticle. The functional group at each end of the linker can show specificity to certain atoms, e.g. -SH, carbohydrate chains, amino, carboxy, thiol groups, or specific end-chains of amino acids, and form linkages that can be cleaved by certain enzymes, e.g. amidases, esterases or acidic environments of the cellular compartments.

In some embodiments, the linker is ((N-[κ-maleimidoundecanoic acid] hydrazide, trifluoroacetic acid salt) (KMUH), mono- or bivalent polyethylene glycols, streptavidin / biotin, . In a preferred embodiment, the linker is KMUH.

The terms "HSA", "HSA protein", "HSA polypeptide", and "HSA sequence" are synonyms and refer to the polymer of amino acid residues disclosed in UniProt P02768 (SEQ ID NO:1), which is human serum albumin, and functional homologues, variants, analogs of fragments thereof. Human serum albumin (Uniprot Accession No. P02768 (ALBU_HUMAN)) is a highly important carrier protein of endogenous and exogenous ligands in plasma. Albumin is a globular protein containing three different structural domains [Carter et al., Adv Protein Chem. 1994;45:153-203; Deeb et al., Biopolymers. 2011;93:161-170]. This protein is an essential carrier protein for hormones, fatty acids, vitamins and drugs [Fanali et al., Mol Aspects Med. 2012;33:209-290].

The sequence for Human Serum Albumin is as follows using the 1-letter amino acid code:

In some embodiments, the HSA polypeptide is a complete HSA sequence. In some embodiments, the HSA polypeptide is a partial HSA sequence.

In some embodiments, the HSA polypeptide comprises an amino acid sequence is at least 40% percent identical, e.g. at least 40%, 45%, 50%, 55%, 60%, 65%, 70%, 75%, 81%, 82%, 83%, 85%, 90%, 91%, 92%, 93%, 94%, 95%, 96%, 97%, 98%, or 99% identical to the sequence SEQ ID NO: 1.

As used herein, the term "size distribution" of the nanoparticle refers to the relative amounts of nanoparticle present in the nanoparticle preparation according to size. In one embodiment, the size distribution of nanoparticles in a nanoparticle preparation is determined by Photon Correlation Spectroscopy (PCS) or transmission electron microscopy (TEM). In a preferred embodiment, the size distribution of nanoparticles comprised in a nanoparticle preparation is determined by PCS.

As used herein, the term "nanoparticle" refers to a particle having a diameter of from 1 to 100 nanometers (nm), having any size, shape, structure or morphology having a surface charge at pH 7 of about zero. In one embodiment, the nanoparticle has a surface charge at physiological pH values (pH 3.0 - 8.5) not exceeding an absolute value of 2.0 mV. In some embodiments, the nanoparticle has a diameter of about 10 - 100 nm, 10 - 80 nm, 15 - 70 nm, 15 - 60 nm, 20 - 60 nm, 20 - 50 nm, 20 - 40 nm, or 30 - 40 nm. In a preferred embodiment, the nanoparticle has a diameter of about 30 - 50 nm. In another embodiment, the nanoparticle has a diameter of about 30 nm.

In one embodiment, the nanoparticle comprises a plurality of molecules, wherein the molecule is preferably a polypeptide. In another embodiment, the molecule is a non-glycosylated polypeptide. In one embodiment, the molecule is not a precious metal.

The term "signaling agent" as used herein refers to any element, molecule, functional group, compound, fragments thereof or moiety that facilitates detection of an agent (e.g., a polysaccharide nanoparticle) to which it is joined. The terms "imaging agent" and "signaling agent" are used interchangeably.

Examples of imaging agents include, but are not limited to: various ligands, radionuclides, fluorescent dyes, chemiluminescent agents, bioluminescent agents, spectrally resolvable inorganic fluorescent semiconductors nanocrystals (e.g., quantum dots, carbon dots), metal nanoparticles (e.g., gold, silver, copper, platinum, etc.), paramagnetic metal ions, enzymes (for specific examples of enzymes, see below), colorimetric labels (such as, for example, dyes, colloidal gold, and the like), biotin, dioxigenin, haptens, and proteins for which antisera or monoclonal antibodies are available. In some embodiments, excited photoluminescence emitters emit within the wavelength range of 300 - 2000nm (this includes both excitation and emission wavelengths). In some embodiments, ultrasound signals arise from reflection of sound waves having frequencies within the range of 10-40 KHz.

The number of signaling agents attached to the nanoparticle determines its capacity to emit amplified signals. For energetically powerful signaling agents such as radioactive nuclides, it is sufficient to attach only one or two agents to a particle for imaging purposes.

In one embodiment of the invention, infrared-emitting agents are used as signaling agent. The absorption characteristics of various human tissues are known in the art. A major factor is the presence of a reduced rate of absorption by water at certain wavelengths (usually stated as about 800-1500 nm) - this is the "water window". It is of advantage if a signaling agent is excited by light at the shorter wavelength end of this "water window", for example at 800 nm, 800 - 850 nm, or 850 - 900 nm. It is of advantage if the signaling agent then emits light at the longer end of the water window, for example at 1500 nm. Although it has been usual in the state of the art to excite photoluminescence emitters at their peak absorption wavelength and to detect their emissions at their peak emission wavelength, recent publications have pointed out that off-peak excitation and detection have become possible due to the much more sensitive detectors now available.

In one preferred embodiment, the signaling agent is proto-porphyrin, in particular proto-porphyrin IX (PP IX). In one preferred embodiment, the signaling agent is PP IX, and the detection of the signal occurs off-peak in order to move its useful spectrum high into the water window.

The present invention may be used in combination with existing therapeutic, diagnostic and imaging techniques. For example, in the screening of the lung for tuberculosis or advanced cancer, the technique in common use should be retained. In the case of lung cancer it is therefore preferable to screen by the usual X-ray imaging, and to modify the screening only be asking the "patient" to inhale our nanoparticles (as aerosol) 10 minutes prior to the X-ray imaging; this minimizes the change experienced by the radiologist and by the "patient" and thus aids introduction of our procedure into the clinic. Of course, the tracheae are close to the body surface and are also very suitable for diagnosis by use of protoporphyrin-based infrared imaging, which involves no exposure to X-irradiation. On this basis our preference will always be to use infrared imaging.

In some embodiments, radiolabels comprise ⁹⁹mTc, ¹¹¹In, ⁶⁴Cu, ⁶⁷Ga, ¹⁸⁶Re, ¹⁸⁸Re, ¹⁵³Sm, ¹⁷⁷Lu, ⁶⁷Cu, ¹²³I, ¹²⁴I, ¹²⁵I, ¹¹C, ¹3N, ¹⁵O, ¹⁸F, ¹⁸⁶Re, ¹⁸⁸Re, ¹⁵³Sm, ¹⁶⁶Ho, ¹⁷⁷Lu, ¹⁴⁹Pm, ⁹⁰Y,²¹²Bi, ¹⁰³Pd, ¹⁰⁹Pd, ¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag, ⁸⁹Zr, and ¹⁹²Ir. In some embodiments, paramagnetic metal ions comprise Gd(III), Dy(III), Fe(III), and Mn(II). In some embodiments, Gadolinium (III) contrast agents comprise Dotarem, Gadavist, Magnevist, Omniscan, OptiMARK, and Prohance. In some embodiments, x-ray reporters comprise iodinated organic molecules or chelates of heavy metal ions of atomic numbers 57 to 83.

In some embodiments, PET (Positron Emission Tomography) tracers are used as imaging agents or signaling agents. In some embodiments, PET tracers comprise ⁸⁹Zr, ⁶⁴Cu, [¹⁸F]fluorodeoxy glucose.

In some embodiments, fluorophores comprise fluorochromes, fluorochrome quencher molecules, any organic or inorganic dyes, metal chelates, or any fluorescent enzyme substrates, including protease activatable enzyme substrates. In some embodiments, fluorophores comprise long chain carbophilic cyanines. In other embodiments, fluorophores comprise Dil, DiR, DiD, and the like. Fluorochromes comprise far red, and near infrared fluorochromes (NIRF). Fluorochromes include but are not limited to a carbocyanine and indocyanine fluorochromes. In some embodiments, imaging agents comprise commercially available fluorochromes including, but not limited to Cy5.5, Cy5 and Cy7 (GE Healthcare); AlexaFluor660, AlexaFlour680, AlexaFluor750, and AlexaFluor790 (Invitrogen); VivoTag680, VivoTag-S680, and VivoTag-S750 (VisEn Medical); Dy677, Dy682, Dy752 and Dy780 (Dyomics); DyLight547, DyLight647 (Pierce); HiLyte Fluor 647, HiLyte Fluor 680, and HiLyte Fluor 750 (AnaSpec); IRDye 800CW, IRDye 800RS, and IRDye 700DX (Li-Cor); and ADS780WS, ADS830WS, and ADS832WS (American Dye Source) and Kodak X-SIGHT 650, Kodak X-SIGHT 691, Kodak X-SIGHT 751 (Carestream Health). In a preferred embodiment, the fluorophore is protoporphyrin IX (PP IX).

### B. Nanoparticle, nanoparticle preparation and composition

As mentioned above, disease diagnoses delayed by failure to detect early lesions result in increased morbidity and mortality, with associated socio-economic costs.

For screening of populations and for monitoring of therapies it is generally sufficient to detect that a lesion is present or absent, since the indication that a lesion may be present serves as clinical indication for further testing, and a simple count of lesions often suffices for monitoring therapy. While the use of nanoparticles to monitor lesions and cancer have been previously attempted, some of these nanoparticles lacked a specific targeting group, and in contrast relied on the so-called enhanced permeability and retention effect (EPR). EPR relates to "passive targeting" and relies on hyperpermeable blood vessels which are biologically present in advanced inflammations or cancers, to access the lesion site. However, since other non-cancerogenic organs may also possess hyperpermeable blood vessels as part of their natural performance, EPR-based nanoparticles are not very specific.

Additionally, the EPR effect was observed in rodent cancer models, but never in humans. Rodent tumours grow much faster, therefore their vessels are much less differentiated than in human tumours. Therefore, nanoparticles which use the EPR effect "target" unspecifically, and they are very likely to fail in the human situation.

Unlike these nanoparticles, the nanoparticles and nanoparticle preparations and compositions of the invention may be used for "active targeting", wherein the nanoparticle comprises a targeting group which is specific for a target site of a tissue.

While early detection methods are known in the art, these methods are more invasive.

There is a need in the art to detect unambiguous signals from lesions with minimal expenditure of resources and minimal discomfort to people. While some methods for early detection are known in the art, the associated costs and side effects are large.

The invention provides a procedure which much enlarges the scope for detecting early-stage disease lesions and of monitoring therapeutic success. The invention further provides compositions that are suitable for use in this procedure.

As an aid to easy comprehension, colon lesions will be used in as an exemplary lesion, which comprises malignant lesions, inflammatory lesions, infections, and lesions in which mixtures of these states are present. However, as explained below the scope of the application is far wider than this example.

In some embodiments, the organic nanoparticle is a nanoparticle comprising a polypeptide.

The invention provides a nanoparticle preparation for molecular imaging or drug delivery, comprising at least one organic nanoparticle having a diameter of less than 100 nm, preferably 10 - 80 nm, more preferably 30 - 60 nm, even more preferably 30nm, wherein about 90% of the organic nanoparticles have a diameter within 25 nm of the average diameter of the nanoparticles of the nanoparticle preparation, wherein the nanoparticle preparation is substantially monodisperse, and wherein the surface charge of the at least one organic nanoparticle at pH 7 is about zero.

In one embodiment, less than 10% of the nanoparticles comprised in the nanoparticle preparation of the invention are unstable, form aggregates and/or have lost their native configuration. In one embodiment, the surface charge of the nanoparticles at pH 7 is about zero. In one embodiment, the surface charge of the nanoparticles at physiological pH values (pH 3.0 - 8.5) does not exceed an absolute value of 2.0 mV.

Living cells typically possess an electrically negatively charged surface coating (glycocalyx). Nanoparticles bearing a negative electrical charge will be repelled, whereas positively charged nanoparticles will be attracted by the negative charge on the glycocalyx. The presence of these electrical forces may cause non-specific adhesion of the nanoparticle to the cell surface or may hinder the approach of the nanoparticle to the vicinity of the target molecule. The nanoparticle of the invention lacks such an electrical charge. In some embodiments, the global surface charge of the inventive nanoparticles resulting from mosaics of small counterbalancing regions of positive and negative charge across the surface, is about zero.

In some embodiments, the nanoparticle is a protein nanoparticle. In another embodiment, the protein nanoparticle is a human serum albumin (HSA) nanoparticle, transferrin nanoparticle, apo-transferrin nanoparticle, streptavidin nanoparticle, granulocyte colony stimulating factor nanoparticle, insulin nanoparticle, or a nanoparticle made from a non-glycosylated or mono-glycosylated protein. In a preferred embodiment, the protein nanoparticle is a HSA nanoparticle, insulin nanoparticle or non-glycosylated protein. In a more preferred embodiment, the protein nanoparticle is a HSA nanoparticle. In some embodiments, the non-glycosylated protein is a de-glycosylated protein.

Without being bound by theory, it is believed that the presence of side-chains on the organic molecules forming the organic nanoparticle, e.g. glycosylated side-chains protruding from polypeptides, could bind non-specifically to surface receptors of the tissue, thereby resulting in undesirable, unspecific signals ("false-positive signals").

The surface charge may be measured using Zeta Sizer as e.g. PSS NICOMPTM 380 DLS/ZLS. Zeta Charge, or Net Surface Charge of a molecule or a particle is measured by PCS in the presence of an applied electrical field.

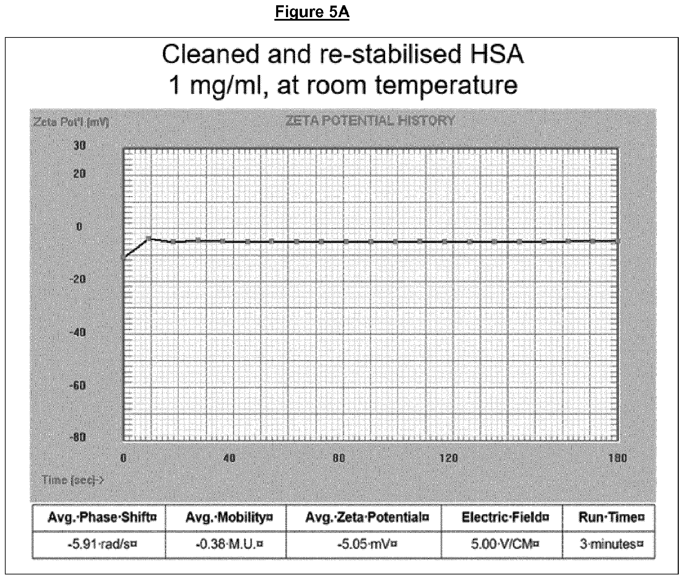

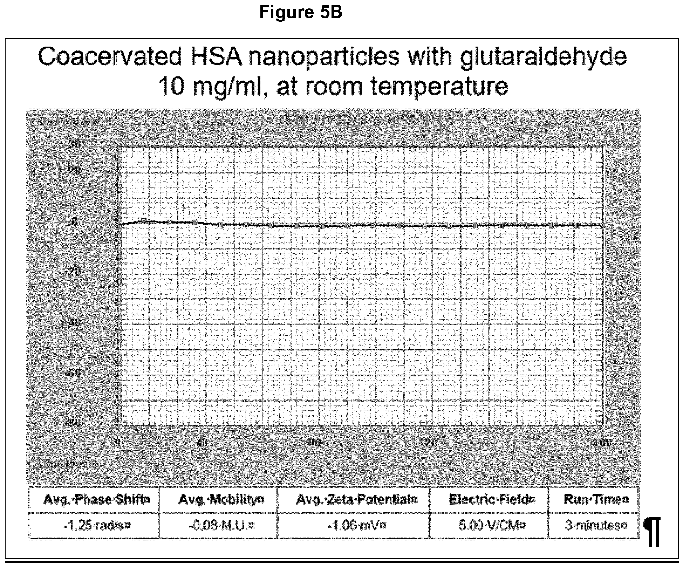

The zeta potential of the nanoparticles disclosed here is close to zero **(****Figure 05****).**

The present invention is not restricted to a specific type of nanoparticle, provided that the nanoparticles can be administered to a cell or a tissue, the surface charge of the nanoparticles at pH 7 is about zero, the surface charge of the nanoparticle at physiological pH values (pH 3.0 - 8.5) does not exceed an absolute value of 2.0 mV, and the size of the nanoparticle is sufficiently small to enable uptake by (normophysiolofical mechanisms of) the target cell.

In one embodiment, the surface charge at pH 7 is neutral or less than about -1 mV. In one embodiment, the surface charge at physiological pH values, i.e. pH 3.0 - 8.5, does not exceed an absolute value of 2.0 mV.

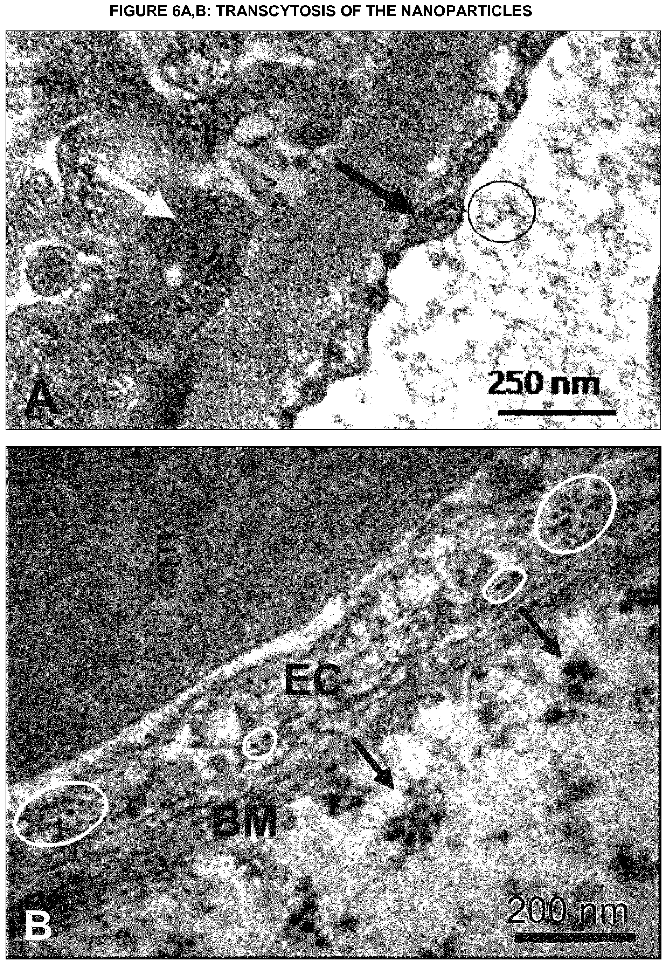

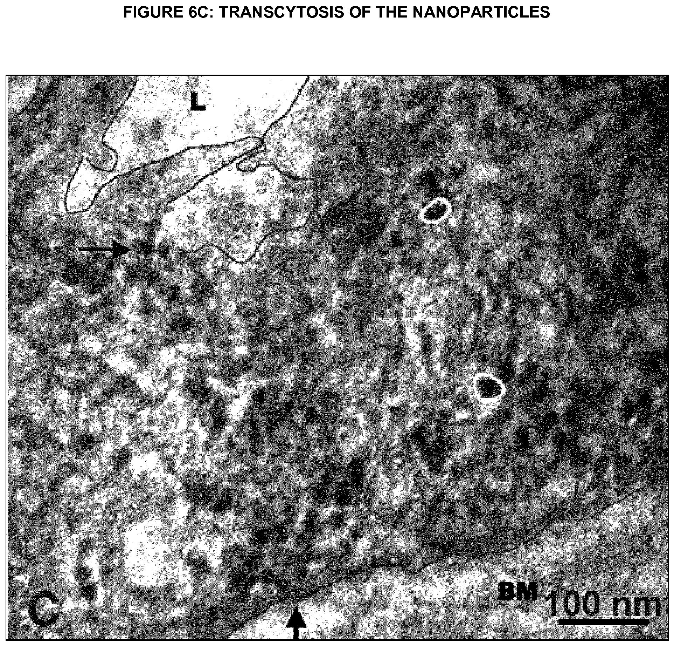

In one embodiment, the nanoparticle is optimized for endocytic uptake by the target cell on the tissue of the subject. For the nanoparticle to be optimized for endocytic uptake by the target cell, the nanoparticle typically has an average diameter of from about 1nm to about 100 nm, more typically from about 2 nm to about 80 nm, from about 10 nm to about 70 nm, from about 15 nm to about 60 nm, from about 20 nm to about 50 nm, or from about 30 nm to about 50 nm. In a preferred embodiment, the nanoparticle has a diameter of about 30 nm. **Figure 06** shows endocytosis of our nanoparticles in various biological systems in living tissues *in vivo* and *ex situ.*

In one aspect, the nanoparticle has a spherical shape.

In one aspect, the nanoparticle preparation has a narrow nanoparticle size distribution. A narrow size distribution is one wherein at least 90% of the nanoparticles comprised in the nanoparticle preparation have a diameter within 25 nm, 20 nm, 15 nm, or 10 nm of the average diameter of the nanoparticles of the nanoparticle preparation. In one embodiment, the at least 90% of the nanoparticles comprised in the nanoparticle preparation have a diameter within 25 nm, 20 nm, 15 nm, or 10 nm of the average diameter of the nanoparticles of the nanoparticle preparation

In one embodiment of the invention, less than 10 %, 9%, 8%, 7%, 6%, 5%, 4%, 3%, 2% or 1% of the nanoparticles comprised in the nanoparticle preparation form aggregates. In one embodiment, less than 5% of the nanoparticles form aggregates. In another embodiment, the nanoparticle preparation is substantially free of aggregates.

In one aspect, the nanoparticle preparation is in form of a liquid, e.g. a dispersion, a suspension, a powder, encapsulation, aerosol or gel.

Provided herein are preparations and compositions comprising organic nanoparticles comprising organic molecules. In some embodiments, the nanoparticle comprises non-glycosylated polypeptides. In some embodiments, the nanoparticle comprises polypeptides that are mono-glycosylated.ln one aspect of the invention, the nanoparticle is a protein nanoparticle, preferably albumin, more preferably human serum albumin (HSA).

In some embodiments, the protein nanoparticle is a human serum albumin (HSA) nanoparticle, streptavidin nanoparticle, insulin nanoparticle, or a nanoparticle made from a non-glycosylated or mono-glycosylated protein. In some embodiments, the protein nanoparticle is a HSA nanoparticle, insulin nanoparticle, or a nanoparticle made from a non-glycosylated or mono-glycosylated protein. In a preferred embodiment, the protein nanoparticle is a HSA nanoparticle. In some embodiments, the non-glycosylated nanoparticle comprises de-glycosylated nanoparticles.

HSA is a highly soluble globular protein with a molecular weight of66.5 kDa and consists of 585 amino acids. HSA is the most abundant protein in the plasma and accounts for 70-80% of the colloid osmotic pressure of human plasma. The amino acid sequence of HSA contains a total of 17 disulfide bridges, one free thiol (Cys 34), and a single tryptophan (Trp 214). While HSA is a preferred protein for making the nanoparticle of the invention, other albumins are contemplated, such as bovine serum albumin. Use of such non-human albumins could be appropriate, for example, in the context of use of these compositions in non-human mammals, such as in veterinary animals (including domestic pets and agricultural animals).

In one aspect, the nanoparticle comprises about 10 - 100 organic molecules forming said nanoparticle. In one embodiment, the organic nanoparticle is a HSA nanoparticle comprising about 10 - 100 HSA molecules per HSA nanoparticle. In one embodiment, the nanoparticle is a HSA nanoparticle comprising about 10 - 20,about 20 - 30, about 30 - 40, about 40 - 50, about 50 - 60,about 60 - 70,about 70 - 80, about 80 - 90, or about 90 - 100 HSA molecules per nanoparticle, while 20 - 30 HSA molecules per nanoparticle is preferred. One possible way of determining the number of HSA molecules in one single nanoparticle is based on the combination of Pyknometry (which is used for determining the density of solutions would give us the density of the whole nanoparticle sample), density of the nanoparticles as measured by TEM, size homogeneity of the nanoparticle preparation, and volume of the spheroidal nanoparticle. The volume of the HSA molecule calculated from a range of methods (small angle neutron scattering, hydrodynamic data, birefringence in an electrical field, frequency dispersion of the dielectric constant, metal-shadowing in electron microscopy, scanning tunneling microscopy) varied from about 110 to about 226 nm³ [Peters, 1996]. HSA molecules, when stirred fast, are thought to arrange themselves like logs in a river. The log-shaped structure of the single HSA molecules will allow them to approach each other very closely. The volume and shape of HSA during the method of the invention is of high importance to be able to calculate the number of HSA molecules in one NP. The nanoparticles of the invention are spheroidal. Assuming that it is in a static crystalloid state, the HSA in the nanoparticles is expected to be an oblate asymmetrical spheroid, so it is unlikely to be packed in the same way as spheres would be, and indeed the packing of oblate asymmetrical ellipsoids can reach significantly higher densities than is possible for spheres (which is 0.74), because the ellipsoids can quasi-tesselate a Euclidean plane, and a number of such planes can in principle be laid over one another to form multi-layer sandwiches. These nanoparticles, which are after coacervation a kind of quasicrystal, packing distances must on the one hand be limited by the crosslinking length of the linking structure, for example glutaraldehyde (∼ 0.7 nm), on the other hand the single HSA molecules therefore have to approach each other at least as close as 0.7 nm for the crosslinking to work. The maximum packing density implies 161 HSA molecules. The largest size calculated for an HSA molecule was about 220 nm³, which would indicate the presence of 64 molecules in one particle.

In some embodiments, the nanoparticle preparation or composition comprises nanoparticles of any shape (e.g., a spherical or non-spherical shape), preferably wherein the nanoparticle is spherical, with an average diameter of no greater than about 50 nm, such as no greater than about any of 45 nm, 40 nm, 35 nm, 30 nm, 25 nm, 20 nm, or 15 nm. In some embodiments, the average diameter of the particles is no greater than about 30 nm. In some embodiments, the average diameter of the particles is between about 20 to about 40 nm. In some embodiments, the average diameter of the particles is between about 20 to about 30 nm. In some embodiments, the particles are sterile-filterable.

### (a) Targeting group

In some embodiments, the above-described nanoparticle further comprises at least one targeting group specific for a target site within an animal or human body, preferably wherein the targeting group is an antibody or fragment thereof. Such targeting groups are useful for targeting the nanoparticle to specific target sites on the tissue of a subject.

The targeting group is capable of specifically binding or specifically targeting to a target on a target cell. The phrase "specifically (or selectively) binds /targets" refers to and encompasses the use of antigen-antibody binding, ligand-receptor binding, and other chemical binding interactions.

Typically, the targeting group is an antibody (e.g., IgG) or a fragment thereof, or other peptide, but can also be a nucleic acid (e.g., DNA,RNA, or an oligonucleotide (aptamer)), a lectin, a specific ligand, or other suitable chemical species.

The targeting group may either be non-covalently or covalently associated with the organic molecule of the nanoparticle, and can be conjugated to the organic molecule by a variety of methods.

Generally, at least one targeting group is attached to the nanoparticle surface. Such attachment may comprise a linker such as, but not limited to, a peptide linker, a disulfide linker, an isothiourea linker, an isourea linker, a sulfonamide linker, an amine linker, a carbamate linker, an amidine linker, a phosphoramidate linker, a thioether linker, an arylamine linker, an arylthioether linker, an ether linker, a hydrazone linker, traizole linker, oxime linker, and combinations thereof.

In one embodiment, the nanoparticle comprises a plurality of targeting groups. In one embodiment, the nanoparticle comprises a plurality of targeting groups, wherein the targeting groups are identical to each other (including monoclonal antibodies), or different to each other (including polyclonal antibodies),. In another embodiment, the nanoparticle preparation or composition comprises a plurality of nanoparticles, wherein the nanoparticles carry a differing targeting groups. In one embodiment, the nanoparticle preparation of the invention comprises a plurality of nanoparticles comprising targeting groups specific for at least two target sites. In one embodiment, the nanoparticle preparation comprises a plurality of nanoparticles comprising attached antibodies specific for at least two target sites on lesions of a tissue, wherein the specific antibodies attached to the nanoparticles enable the determination of the cancer stage or cancer progression. This "nanoparticle cocktail" will provide a high level of specificity for a specific target tissue.

In one embodiment, the targeting group is associated with the nanoparticle through non-covalent bonds. In one embodiment, the targeting group is associated with the nanoparticle through covalent bonds.

In one embodiment, the targeting group is an antibody or a fragment thereof. In one embodiment, the targeting group is a monoclonal or polyclonal antibody. In one embodiment, the antibody is non-covalently associated with the nanoparticle via the antibody-binding motif. In one embodiment, the antibody is non-covalently associated with the nanoparticle via an albumin-binding motif. In one embodiment, the nanoparticle is avidinylated and at least one biotinylated monoclonal antibody is attached to the nanoparticle non-covalently, e.g. electrostatically. In one embodiment, the at least one biotinylated monoclonal antibody is a plurality of biotinylated monoclonal antibodies. In one embodiment, at least a subset of the nanoparticles comprises more than one antibody-binding motif.

In a preferred embodiment, the nanoparticle is a HSA nanoparticle, comprising an anti-EpCAM antibody as a targeting group, enabling the specific attachment of the nanoparticle to EpCAM-expressing lesion sites in the tissue. In one embodiment, the EpCAM antibody is a monoclonal antibody.

The invention further provides a composition comprising the nanoparticle preparation of the invention. In one embodiment, the composition further comprises a pharmaceutically acceptable excipient.

In some embodiments, the targeting group is attached to the organic molecule before the nanoparticle is formed by the method of the invention. In other embodiments, the targeting group is attached to the nanoparticle after the method of the invention has produced the nanoparticle.

In some embodiments, the targeting group may be either anchored in the hydrophobic portion of the organic molecule or attached to reactive groups of the organic molecule, preferably in a hydrophilic region of the organic molecule. The targeting group may be attached to the nanoparticle via a linker to a reactive group. Preferred reactive groups include amino groups, carboxylic groups, hydrazide groups, and thiol groups. The coupling of the targeting group to the organic molecule can be performed by standard methods of organic chemistry that are known to those skilled in the art.

In certain embodiments, the nanoparticle comprises about 1 targeting group per nanoparticle to about 20 targeting groups per nanoparticle.

Targeting groups are any ligand specific for a characteristic component, i.e. the target site, of the targeted region. Preferred targeting groups include proteins such as polyclonal or monoclonal antibodies, antibody fragments, or chimeric antibodies, enzymes, or hormones, or sugars such as mono-, oligo- and poly-saccharides. It is contemplated that the antibodies as would be known to the person skilled in the art, may be used to target the nanoparticles of the present invention to specific tissues and cell types. In certain embodiments of the invention, contemplated targeting groups interact with a target site, wherein said target site may be an integrin, proteoglycan, glycoprotein, receptor or transporter, preferably on the surface of the tissue facing a body cavity, wherein the expression level of such a target site at the site of the lesion is significantly higher than in a non-lesion tissue. Suitable targeting groups include any that are specific for cells of the target organ, or for structures of the target organ exposed to a body cavity, and which are differentially expressed due to local pathology, such as an inflammation or tumor.

In some embodiments, a targeting group will be chosen to correspond to a target site, wherein the target is a receptor specifically expressed on the target cell population. The organic nanoparticle to be delivered will bind specifically to a target site on a target cell, and deliver the contents, such as the signaling agent or active agent, to a cell. In some preferred embodiments, the target site on the target cell is a molecule that is overexpressed relative to the non-pathological tissue surrounding the pathological target cells. The level of overexpression may be between about 2-fold to about 500-fold relative to the expression level of the corresponding non-pathological tissue. In other embodiments, the level of overexpression of the target site is about 2-fold to about 450-fold, about 5-fold to about 400-fold,about 10-fold to about 300-fold,about 15-fold to about 250-fold,about 20-fold to about 200-fold,about 30-fold to about 150-fold,about 40-fold to about 100-fold,about 50-fold to about 90-fold,about 60-fold to about 85-fold,or about 70-fold to about 80-fold.

In some embodiments, suitable targeting groups are antibodies or antibody fragments, peptides, oligonucleotides for example in the form of aptamers, nucleotides, specific ligands, lectins.

A skilled person realizes that the systems and methods of the present invention can be employed in a variety of types of experimental, imaging, therapeutic and diagnostic procedures, including in vitro or in vivo experimental procedures. The systems and methods of the present invention can be applied to a cell or a tissue, wherein the cell can be part of a tissue, such as an inflammatory tissue or tumor tissue.

### (b) Target sites

The nanoparticles of the invention specifically bind to a target site on a tissue. Many possible target sites are known in the art that are specific for inflammatory lesions, or various stages of tumor development, infection or any combination thereof.

The target site must be present in the target cell, i.e. the pathological cell, at a higher concentration than the surrounding, non-pathological tissues. In on embodiment, the target site is the Epithelial Cell Adhesion Molecule (EpCAM). EpCAM is typically expressed on a non-pathological, normal epithelial cells at the basolateral aspect of the cell and is not available for binding of nanoparticles at the apical aspect. In adenocarcinoma cells EpCAM expression is upregulated in amount and EpCAM also appears in substantial amounts apically [Ogura et al., 19998; Trzpis et al., 2007; Xia et al., 2005], and is thus available to bind nanoparticles. Thus EpCAM profiles provide a natural contrast to the surrounding tissues, outlining the lesion with strong contrast. This distinct lesion-specific expression level is utilized by the nanoparticles of the invention, and rendered into a strong lesion-specific signal that can be detected even before any gross morphological abnormalities present themselves. As the dysplastic epithelial cells enter the epithelial-mesenchymal transition, EpCAM expression is downregulated, making EpCAM a stage-specific target molecule.

Moreover, since various molecules exhibit a specific temporal and spatial overexpression during the different stages of tumor development and/or inflammation, it is possible to determine the specific stages of a tumor, e.g. pre-cancerous hyperplastic epithelium, or early-stage carcinoma, by assessing the expression level and pattern of these known molecules.

As an example, EpCAM is initially overexpressed in hyperplastic epithelia, but does not survive the Epithelial-Mesenchymal-Transition ("EMT") that occurs when a transformed epithelial cell breaks through the basement membrane of its epithelial sheet and becomes amoeboid (mesenchymal characteristic). In this case, the anti-EpCAM targeted nanoparticle will not bind to the malignant cells, thus no signal will be detected. However, the anti-EpCAM targeted nanoparticle will specifically detect the *early stages* of malignant transformation, but not the later stages, thereby allowing the early detection of an epithelial lesion before the tissue could develop into a malignant tumor. Choosing the right target site, and thus targeting group for attaching to the nanoparticle, will allow elucidation of the presence and severity of any epithelial lesion.

In the case of living cells, the stable, attachment of a ligand at the cell surface triggers endocytotic mechanisms leading to internalization of the targeting group ("ligand") in an endosome, which represents a membrane-bounded bubble of the external medium within the cytoplasm of the cell, and which is part of the clearing mechanisms of the cell itself. Endocytosis of materials has been carefully studied and is largely understood. Endosomes undergo a cycling process, resulting in the return of the "target" molecule, e.g. EpCAM, to the cell surface (during a period of hours). The targeting group, i.e. the ligand bound to the target site on the cellular surface, is processed by the lysosomal machinery of the cells, where it will be deposited within about 15 minutes in a terminal lysosome which will eventually degrade it.

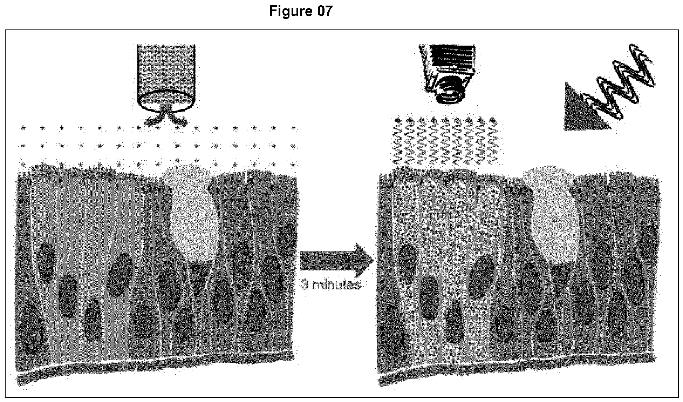

In some embodiments, the trafficking of the targeting group through the endo-lysosomal system will transfer the nanoparticle into the terminal lysosomes if the connection between the nanoparticle body and the targeting group is stable to pH changes, for example if it is covalently bound. The result of endocytosis of the nanoparticle is to create a depot of the nanoparticle throughout the cytoplasm of the target cell and thus to raise the number of nanoparticles in a given area of the lesion that can generate signals. The increase is significant because the signal no longer derives from a surface (for example 5 µm²) but from a volume (for example 5x15 = 75 µm³). The size and volume of the cell determines the magnitude of this effect. In addition to this, the uptake of the nanoparticles into the target cells increases the effective signal contrast between the lesion and the normal surrounding tissue, which does not take up the nanoparticles. **Figure 07** visualizes the increase in effective contrast in an idealized screening arrangement.

The coexistence of different pathophysiological conditions within a lesion is common. Carcinomas frequently originate in chronically inflamed tissues, and within a carcinoma there is a natural progression from stage to stage within the tumor, and indeed from stage to stage across the tumor surface, where actively migrating metastatic cells may be most common at the tumor edge where they are migrating along blood vessels. The lesion will generate stronger signals if the full range of pathologies within it binds nanoparticles. If necessary, the distribution of the various pathophysiological regions within the lesion can be examined in subsequent examinations, after the lesion has been detected.

In some aspects of the invention, the nanoparticles remain stable the target cells for a time period long enough to detect signals. The nanoparticles should preferably resist breakdown in the lysosomal compartment of the target cells for this period of time, which is achievable by preparing the nanoparticle according to the inventive method provided herein. The cross-linked organic nanoparticles extend the degradation process to several hours, thereby allowing a large time period for detecting the signal emitted by the nanoparticles taken up by the target cells.

In one embodiment, the target site is on an inflammatory lesion, a tumor antigen, a stage-specific tumor antigen, a dysplastic epithelium, an early-stage carcinoma, a metastatic malignancy, an infectious antigen such as a viral antigen, bacterial antigen, fungal antigen, protozoan antigen, or worm antigen, a pre-cancerous lesion, or a cancerous lesion.

In one embodiment, the target site is selected from the group consisting of epithelial cell adhesion molecule (EpCAM), Cell Adhesion Molecules (CAMS), E-cadherin, cytokeratins pan-cytokeratins, collagens, elastin, keratin, fibronectin, Tenascin-C, integrins, α5β6 integrin, MMPs, perlecan, vimentin, E- Cadherin, N-Cadherin, desmoglein, desmoplakin, plakoglobin, catenin, Fas/Fas ligand, Bacteria (syphilis), Fungus (Candida),T-cell receptor, Marker for transition from dysplasia (adenoma) to carcinoma: PROX-1, Growth Factor Receptors for: EGF, IGF, BMP, TGF-β,PI3K/AKT, TGF-β, ALK3, Leptin receptor, Bone Morphogenetic proteins (BMPs), Cyclin D1 (CCND1), CDC6, CDC45, CDT1, MCM2, MCM3, MCM4, MCM5, MCM6 and MCM7, Claudins, Cldn2, Cldn4 and Cldn8, PTCH1, zinc finger protein gut-enriched Kruppel-like factor (GKLF)/epithelial zinc finger, HPV, Epstein-Barr, CD3, CD8, CD4, CD28, CD80, CD103, CD105, ###, or target molecules characteristic of the viruses Adenoviridae spp., Herpesviridae spp., Papillomaviridae spp., Polyomaviridae spp., Poxviridae spp., Hepadnaviridae spp., Parvoviridae spp., Astroviridae spp., Caliciviridae spp., Picornaviridae spp., Coronaviridae spp., Flaviviridae spp., Togaviridae spp., Hepeviridae spp., Retroviridae spp., Orthomyxoviridae spp., Arenaviridae spp., Bunyaviridae spp., Filoviridae spp., Paramyxoviridae spp., Rhabdoviridae spp., Reoviridae spp., and the Hepatitis D virus, and target molecules characteristic of the bacteria: Bacillus spp., Bartonella spp., Bordetella spp., Borrelia spp., Brucella spp., Campylobacter spp., Chlamydia spp. and Chlamydophila spp., Clostridium spp., Corynebacterium spp., Enterococcus spp., Escherichia spp., Francisella spp., Haemophilus spp., Helicobacter spp., Legionella spp., Leptospira spp., Listeria spp., Mycobacterium spp., Mycoplasma spp., Neisseria spp., Pseudomonas spp., Rickettsia spp., Salmonella spp., Shigella spp., Staphylococcus spp., Streptococcus spp., Treponema spp., Ureaplasma spp., Vibrio spp., Yersinia spp., and target molecules characteristic of the fungal genera: Candida spp., Aspergillus spp., Blastomyces spp., Coccidioides spp., Cryptococcus spp., Fusarium spp., Histoplasma spp., Mucor genera, Rhizopus genera, Absidia genera, Actinomycetoma spp., Eumycetoma spp., Pneumocystis spp., Microsporum spp., Trichophyton spp., Epidermophyton spp., Sporothrix spp., Paracoccidioides spp., Talaromyces spp., and Stachybotrys spp.

In a preferred embodiment, the target site is selected from the group consisting of epithelial cell adhesion molecule (EpCAM), CD22, CD44, MUC1, LMP2, surviving, androgen receptor, cyclin B1, EFG-R, PLAC1, HER-2neu, polysialic acid, mesothelin, PSCA, carbonic anhydrase IX, VEGF-R1, VEGF-R2, PSA, Pro-PSA, mPSA, BRCA-1, and BRCA-2. In a more preferred embodiment, the target site is EpCAM. In another embodiment, the target site is one selectively expressed or overexpressed during cancer and/or inflammation.

### (c) Signaling agent

In some embodiments, the nanoparticle preparation comprises a nanoparticle further comprising a signaling agent attached to the nanoparticle. In one embodiment, the signaling agent is preferably selected from the group consisting of photoluminescence agents, ultrasound emitters, microwave and radio-wavelength emitters, radionuclides, fluorescent dyes, chemiluminescent agents, bioluminescent agents, spectrally resolvable inorganic fluorescent semiconductors nanocrystals and quantum dots. In a preferred embodiment, the signaling agent is protoporphyrin IX (PP IX).

In some embodiments, the signaling agent is comprised in the inner portion of the nanoparticle, i.e. the signaling agent is not attached to the outside of the nanoparticle. By avoiding the attachment of the signaling agent to the outside of the nanoparticle of the invention, any possible non-specific binding of the nanoparticle due to the signaling agent is minimized, particularly if the signaling agent bears an electrical charge. Further, attaching the signaling agent on the surface of the nanoparticle would render the nanoparticle much larger, thereby reducing the probability of successful uptake of the nanoparticle into the cell by endocytosis. Thus, for some embodiments, the ideal position for signaling agents is inside the nanoparticle.

In some embodiments, each organic molecule is conjugated with the maximum possible number of signaling agents, in order to maximize the signal strength obtainable from each nanoparticle. In some embodiments, the organic molecule, such as HSA, is attached with 1, 2, 3, 4, 5, 6, 6, 7,8, 9, or 10 signaling agents. In a preferred embodiment, the organic molecule, such as HSA, is attached with various ligands, radionuclides, fluorescent dyes, chemiluminescent agents, bioluminescent agents, spectrally resolvable inorganic fluorescent semiconductors nanocrystals (e.g., quantum dots, carbon dots), metal nanoparticles (e.g., gold, silver, copper, platinum, etc.), paramagnetic metal ions, enzymes (for specific examples of enzymes, see below), colorimetric labels (such as, for example, dyes, colloidal gold, and the like), biotin, dioxigenin, haptens, and proteins for which antisera or monoclonal antibodies are available. In some embodiments, excited photoluminescence emitters emit within the wavelength range of 300 - 2000nm (this includes both excitation and emission wavelengths). In some embodiments, ultrasound signals arise from reflection of sound waves having frequencies within the range of 10-40 KHz.

In some embodiments, after the nanoparticle has specifically bound to and taken up by the target cell, i.e. the lesion, the lesions can be detected due to signals emanating from the nanoparticles.

The skilled person will know which appropriate detector must be employed and to use an appropriate stimulator. The present state of the art employs a range of different technologies to generate and detect signals from nanoparticles.

For the present invention, the signaling agent should be chosen to minimize any harm to the subject, that might be caused by the ionization damage. In some embodiments, the electromagnetic and mechanical forces resulting from signal generation from the nanoparticles of the invention are about zero, or as close to zero as possible. The preferred signalling agents of this invention do not ionize tissue components, so that nuclides and X-rays are not preferred. Radio waves, visible light and infrared light waves, magnetic fields, sound waves are non-ionizing and therefore preferred. Most preferred are infrared light waves between 700-20,000 nm wavelength.

Two exemplary detection modes are provided herein. In the case, wherein the material between the signal-emitting lesion containing the nanoparticle, and the detector is mostly a fluid, e.g. during endoscopy or colonoscopy, the space between the lesion and the detector is small, e.g. a few centimeters or less. In this favorable circumstance, signal energies of micro-electron volt levels are sufficient to provide highly reliable detection. One preferred solution in this case is to employ electromagnetic energies in the visible or near-infrared spectrum by using light of wavelength 700 nm to 20,000 nm (Thurner & Debbage, 2018).

Alternatively, if the material between the signal-emitting lesion and the detector comprises not only fluids, but also tissues and/or minerals, e.g. during detection from outside the body, significant amounts of fluids in and around living tissues are interposed between the nanoparticle and detector. In such cases, signal energies of millivolt levels are required to provide highly reliable detection. The required energy levels may be calculated by the formula discussed by (Thurner & Debbage, 2018), which is incorporated herewith. We have previously published a formulation that quantitates the energies required for signal generation and detection along the path between the stimulator and the lesion and along the return path between the lesion and the detector (Thurner & Debbage, 2018).

The detection of lesion emitting signals due to the presence of targeted nanoparticles can be carried out from outside the body for example by positioning the detector on the surface of the body at different points, and moving the detector through a range of angles at those points, in a manner identical to the fashion in which ultrasound examinations are carried out. This permits both detection and localization of lesions to within a few centimeters, which is sufficient to serve as a clinical indication for further testing to be carried out in order to obtain a diagnosis.

### (d) Active agents

In one embodiment, the nanoparticle comprises an active agent. In one embodiment, the nanoparticle comprises an active agent bound to the nanoparticle. In one embodiment, the active agent is an anti-cancer agent. In another embodiment, the active agent is an antiinflammatory agent.

In some embodiments, the nanoparticles comprise an active agent in the core of the nanoparticle. In some embodiments, the nanoparticles comprise a plurality of different therapeutic agents in the nanoparticle of the invention.

In some embodiments, the active agent is a chemotherapy drug, antimetabolite agents (including nucleoside analogs), a cytotoxic agent (e.g. a toxin), alkylating agents, tyrosine kinase inhibitors, anthracycline antibiotics, vinca alkloids, proteasome inhibitors, taxanes, modulators of HER2/neu, modulators of EGFR, farnosyltransferase inhibitors, and topoisomerase inhibitors.

The active agent described herein can be the agent itself, or a pharmaceutically acceptable salt thereof, pharmaceutically acceptable ester thereof, as well as stereoisomer, enantiomer, racemic mixture, and the like.

The nanoparticles described herein may be present in a dry formulation (e.g., lyophilized composition) or suspended in a biocompatible medium. Suitable biocompatible media include, but are not limited to, water, buffered aqueous media, saline, buffered saline, optionally buffered solutions of amino acids, optionally buffered solutions of proteins, optionally buffered solutions of sugars, optionally buffered solutions of vitamins, optionally buffered solutions of synthetic polymers, lipid-containing emulsions, and the like.

### C. Preparation method

In a further aspect, the invention relates to a method of preparing a nanoparticle preparation comprising organic nanoparticles having a diameter of less than 100 nm, preferably 10 - 80 nm, more preferably 30 - 60 nm, even more preferably 30nm, wherein about 90% of the organic nanoparticles have a diameter within 25 nm, within 20 nm, within 15 nm, within 10 nm, or within 5 nm of the average diameter of the organic nanoparticles comprised in the nanoparticle preparation, wherein the nanoparticle preparation is substantially monodisperse, and wherein the surface charge of the organic nanoparticles at pH 7 is about zero.

The method comprises
a) Providing an aqueous solution of an organic molecule in its native conformation at pH 4 to pH 8, and at a concentration of about 1 mg/ml to about 50 mg/ml, wherein the organic molecule is preferably a protein, more preferably HSA;
b) Adding an inorganic salt, preferably an alkali salt, more preferably sodium chloride, to a final concentration of 0.5 to 2 M under continuous stirring and at a temperature of about 40 about 65 °C for a time period sufficient to enable coacervate formation;
c) Cooling the solution to ambient temperature; and
d) Adding a cross-linking agent at a final concentration of about 0.05 %(w/v) to 0.2 % (w/v), preferably wherein the final concentration is 0.1 % (w/v), and further wherein the cross-linking agent is preferably glutaraldehyde, 1,5-dichloropentane or glutaryl chloride.

In one embodiment, the cross-linking agent is glutaraldehyde.

In one embodiment, the organic molecule in its native conformation is cleaned and stabilized. In one embodiment, the nanoparticle comprises at least one polypeptide. In one embodiment, the polypeptide is human serum albumin (HSA).

In a preferred embodiment, the method of preparing a HSA nanoparticle comprises:
a) Providing a stabilized HSA sample at pH 4 - pH 8, and at a concentration of about 1 mg/ml to about 50 mg/ml;
b) Adding an inorganic salt, preferably an alkali salt, more preferably sodium chloride, to a final concentration of 0.5 to 2 M under continuous stirring and at a temperature of about 40 - about 65 °C for a time period sufficient to enable coacervate formation;
c) Cooling the solution to ambient temperature;
d) Adding a cross-linking agent Adding a cross-linking agent at a final concentration of about 0.05 %(w/v) to 0.2 % (w/v), preferably wherein the final concentration is 0.1 % (w/v); and
e) Incubating the sample overnight at room temperature under stirring.

In another embodiment, the method of preparing a HSA nanoparticle comprises:
a) Providing a cleaned and stabilized HSA solution in a concentration of about 15 mg/ml, at a pH of about 6.8 - 7.2;
b) Adding NaCl to a final concentration of 1 M;
c) Heating the solution to about 50°C under fast stirring for a time period sufficient to coacervate formation;
d) Cooling the solution to room temperature while continuously stirring as in step (c);
e) Adding a 1% glutaraldehyde solution in a ratio of about 1:10 (v/v) relative to the reaction solution while lowering the stirring to moderate speed;
f) Incubating the solution overnight at room temperature under stirring.

In another embodiment, the method of preparing a HSA nanoparticle comprises:
a) Providing a cleaned and stabilized HSA solution in an aqueous solution in a concentration of about 10 to about 15 mg/ml, at a pH of about 6.8 - 7.2;
b) Adding of NaCl to a final concentration of 1M ;
c) Heating the solution to about 50°C under stirring for a about 5 hours;
d) Cooling the solution to room temperature while continuously stirring as in step (c);
e) Adding a 1% glutaraldehyde solution in a ratio of about 1:10 (v/v) relative to the reaction solution while lowering the stirring to moderate speed;
f) Incubating the solution overnight at room temperature under stirring.

In general, the time period sufficient to enable coacervate formation is less than 10 hours. In some embodiments, the time period sufficient to enable coacervate formation is from about 1 to about 10 hours, from about 2 to about 9 hours, from about 3 to about 8 hours, from about 4 to about 7 hours, from about 4 to about 8 hours, from about 4 to about 7 hours, or from about 4 to about 6 hours. In a preferred embodiment, the time period sufficient to enable coacervate formation is from 4.5 to about 5.5 hours, more preferably about 5 hours.

In some aspects of the inventive methods, the pH of the sample or aqueous solution comprising the organic molecule is about pH 6.8 - 7.2. In other embodiments, the pH is about pH 6.8 to about 7.1, or about pH 6.9 - about 7.1. In a preferred embodiment, the pH is neutral, i.e. about pH 7.0.

In some embodiments, the inorganic salt, preferably an alkali salt, more preferably sodium chloride, is added under continuous stirring of the solution comprising the organic molecule. In some embodiments, the stirring is performed at a speed of about 300 to about 600 rpm, about 350 to 550 rpm, or about 400 to about 500 rpm. In a preferred embodiment, the stirring is performed at a speed of about 400 rpm. In some embodiments, the stirring step is performed for about 4 to about 6 hours, preferably from 4.5 to about 5.5 hours, more preferably for about 5 hours.

In some embodiments, the above recited heating and cooling steps to room temperature are also performed under continuous stirring of the solution. In some embodiments, the stirring is performed at a speed of about 300 to about 600 rpm, about 350 to 550 rpm, or about 400 to about 500 rpm. In a preferred embodiment, the stirring is performed at a speed of about 400 rpm. In some embodiments, the stirring speed is the same during adding the inorganic salt and the heating and cooling step.

In some embodiments, the addition of the cross-linking agent, preferably glutaraldehyde, as and/or the step of incubating the solution overnight at room temperature, occur under continuous stirring. In some embodiments, the stirring is performed at a speed of about 100 to about 300 rpm, about 150 to 250 rpm, more preferably about 200 rpm. In some embodiments, the stirring speed is the same during adding the cross-linking agent and the overnight incubating step at room temperature.

The term "rpm" as used herein refers to "revolutions per minute", wherein the stirring speed of 100 to 1000 rpm is indicated for a vessel diameter of about 10 cm. The corresponding rpm parameters for a smaller or larger vessel are within the general knowledge of the skilled person.

In some aspects of the inventive methods, the concentration of the organic molecule in the sample or aqueous solution is between 1 mg/ml to 50 mg/ml. In other embodiments, the concentration of the sample or aqueous solution is about 2 mg/ml to about 48 mg/ml, from about 3mg/ml to about 45mg/ml, from about 4mg/ml to about 40mg/ml, from about 5mg/ml to about 38mg/ml, from about 6mg/ml to about 35mg/ml, from about 7 mg/ml to about 32mg/ml, from about 8mg/ml to about 30 mg/ml, from about 9 mg/ml to about 28 mg/ml, from about 10 mg/ml to about 25mg/ml, or from about 10mg/ml to about 22mg/ml. In a preferred embodiment, the concentration of the organic molecule in the sample or aqueous solution is about 10 mg/ml to about 20 mg/ml, preferably from about 12mg/ml to about 18mg/ml, more preferably from about 14mg/ml to about 16 mg/ml, most preferably about 15 mg/ml.

In some aspects of the inventive methods, the concentration of the inorganic salt added at step (b) of the inventive method, resulting in a concentration of 0.5 to 2 M. In other embodiments, the final concentration of the inorganic salt in the solution is from about 0.5 M to 1.9 M, from about 0.6 M to 1.8 M, from about 0.7 M to 1.7 M, from about 0.8 M to 1.6 M, from about 0.9 M to 1.5 M, from about 1.0 M to 1.4 M, from about 1.1 M to 1.3 M, from about 1.1 M to 1.2 M. In a preferred embodiment, the final concentration of the inorganic salt in the solution is about 0.7 M to 1.3 M, more preferably from about 0.8 M to 1.2 M, more preferably from about 0.9 M to 1.1 M, and most preferably about 1 M.

Suitable inorganic salts for step (b) of the inventive method comprise alkali metals.

In some aspects of the inventive methods, the inorganic salt is sodium chloride.

In some aspects of the inventive methods, step (b) of the inventive methods is performed at a temperature of about 40 - 65 °C. In other embodiments, the temperature is from about 42 °C to about 63 °C, from about 44 °C to about 61 °C, from about 46 °C to about 60 °C, from about 48 °C to about 58 °C, from about 50 °C to about 56 °C, from about 51 °C to about 55 °C, from about 52 °C to about 54 °C, or from about 53 °C to about 54 °C. In a preferred embodiment, the temperature is from about 45 °C to about 55 °C, from about 46 °C to about 54 °C, more preferably from about 47 °C to about 53 °C, from about 48 °C to about 52 °C, even more preferably from about 49 °C to about 51 °C, or most preferably at a temperature of about 50 °C.

Suitable cross-linking agents used in the inventive methods are compounds which form linkers between the organic molecules of the nanoparticle, thereby forming and stabilizing the organic nanoparticle. In some embodiments, the cross-linking agent is glutaraldehyde, 1,5-dichloropentane or glutarylchloride, preferably wherein the cross-linking agent is glutaraldehyde. In some embodiments, the cross-linking agent comprises 15, 14, 13, 12, 11, 10, 9, 8, 7, 6, 5, 4, or 3 carbon atoms.

In some aspects of the inventive methods, the cross-linking agent recited in step (e) of the inventive methods is present at a concentration of about 0.05% (w/v) to about 0.2% (w/v) in the nanoparticle reaction mixture. In some embodiments, the cross-linking agent is present at a concentration of about 0.05% (w/v) to about 0.06% (w/v), about 0.06% (w/v) to about 0.07% (w/v), about 0.07% (w/v) to about 0.08% (w/v), about 0.08% (w/v) to about 0.09% (w/v), about 0.09% (w/v) to about 0.1% (w/v), about 0.11% (w/v) to about 0.12% (w/v), about 0.12% (w/v) to about 0.13% (w/v), about 0.13% (w/v) to about 0.14% (w/v), about 0.14% (w/v) to about 0.15% (w/v), about 0.15% (w/v) to about 0.16% (w/v), about 0.16% (w/v) to about 0.17% (w/v), about 0.17% (w/v) to about 0.18% (w/v), about 0.18% (w/v) to about 0.19% (w/v), or about 0.19% (w/v) to about 0.2% (w/v). In a preferred embodiment, the cross-linking agent is present at a concentration of about 0.08% (w/v) to about 0.12% (w/v), more preferably at a concentration of about 0.1% (w/v).

In one embodiment, the nanoparticle preparation does not comprise, i.e. lacks, precious metals. In one embodiment, the nanoparticle preparation is stable in the sense that the organic nanoparticles do not form a substantial amount of aggregates. In a preferred embodiment, the nanoparticle is a stable HSA nanoparticle. In a preferred embodiment, the nanoparticle preparation comprises organic nanoparticles wherein the surface charge of the organic nanoparticles at physiological pH values, i.e. pH 3.0 - 8.5, does not exceed an absolute value of 2.0 mV, 1.5 mV, 1.0 mV, or 0.5 mV.

In some embodiments, the method of the invention may comprise a step of diluting or concentrating the prepared organic nanoparticle to a nanoparticle preparation having a desired concentration. In some embodiments, the method of the invention may further comprise a step of lyophilizing the prepared organic nanoparticle.

As described herein, the inventive preparation methods allow the manufacture of an advantageous nanoparticle preparation comprising organic nanoparticles having a diameter of less than about 100 nm, preferably about 10 - about 80 nm, more preferably about 30 - about 60 nm, even more preferably about 30 nm, wherein at least 90% of the organic nanoparticles have a diameter within about 25 nm, about 20 nm, about 15 nm, about 10 nm, or within about 5 nm of the average diameter of the organic nanoparticles comprised in the nanoparticle preparation, wherein the nanoparticle preparation is substantially monodisperse, and wherein the surface charge of the organic nanoparticles at pH 7 is about zero. In a preferred embodiment, the above nanoparticle preparation comprises organic nanoparticles having a diameter of about 30 - about 60 nm, preferably about 30 nm, wherein at least 90% of the organic nanoparticles have a diameter within about 10 nm, preferably within about 5 nm of the average diameter of the organic nanoparticles comprised in the nanoparticle preparation. This nanoparticle preparation overcomes the shortcomings of the nanoparticles of the prior art, which usually display a) a wide size distribution, (b) comprise charged nanoparticles, and c) are not stable and form aggregates in solution.

The avoidance of such unwanted side effects associated with charged, non-homogenous nanoparticle preparations entails many advantages. For example, unlike other nanoparticle compositions currently on the market, the inventive nanoparticle preparation and composition has greater stability, e.g. the nanoparticle preparation does not form substantial amounts of aggregates, but remains stable, e.g. in dispersion, suspension or powder form, for at least about 1 month, about 3 months, about 6 months, about 9 months, about 12 months, about 18 months, about 24 months or longer when stored at about 4 °C. Further, the inventive nanoparticle preparation and composition has a narrow size distribution of organic nanoparticles, wherein the size of the nanoparticles allows uptake of the nanoparticles into the target cells. Without being bound by theory, it is believed that the uptake occurs into the target cell by endocytosis or endocytosis-like mechanisms. This allows inter alia the active therapeutic agent, active agent or signaling agent to be delivered specifically into the target cells, allowing the specific imaging and/or treating of the target cell.

Without being bound by theory, it is believed that the combination of the stirring and temperature in the presence of a high concentration of inorganic salt allows the formation of uniquely uniform nanoparticles of small size. It is believed that the stabilized organic molecules, such as HSA, are present in the solution in its native conformation and shape. The stirring induces the organic molecules to realign. The warm temperature and high concentration of the inorganic salt will camouflage the charge of the organic molecules, so that they can approach each other very closely. The cross-linking agent then links the organic molecules together, resulting in uniformly sized and stable nanoparticles.

The nanoparticle obtained by the above method can be analyzed by assays known in the art, including but not limited to Photon Correlation Spectroscopy (PCS), Electron Microscopy (EM), Thin Layer Chromatography (TLC), Gel-Electrophoresis, density of fixed particles, Pierce Bicinchoninic Acid Assay (BCA assay), Zeta Potential, tests on different functional groups (e.g. COOH / NH₂ / SH groups), HSA-Immunohistochemistry (IHC), Ultraviolet Spectroscopy (UV Spectroscopy)..

Each of the mentioned methods has its advantages and its drawbacks, it is therefore optimal to run two or more of the methods in parallel, as we did with PCS and EM.

### (a) Stabilization of organic molecule

In some embodiments, any of the methods disclosed herein may further comprise the step of stabilising the organic molecule prior to the generation of the nanoparticle.

An exemplary stabilization method comprises
a) Providing a solution comprising the organic molecule at a pH of around 4 - 5;
b) Adding an inorganic salt, preferably sodium chloride to a concentration of about 150 mM;
c) Adding a fatty acid, preferably sodium caprylate;
d) Adjusting the pH to about 10 and incubating the solution for about 1 hour at room temperature;
e) Adjusting the pH to about 7, and further incubating the solution overnight.

The stabilization provides the organic molecule in a native conformation, and thus supports the nanoparticle formation.

In one embodiment, the stabilization of the organic molecule comprises the addition of a fatty acid to said organic molecule. In another embodiment, the fatty acid is mixed with the organic molecule, preferably HSA, prior to step (a) above. In one embodiment, the fatty acid is a saturated or unsaturated fatty acid. In another embodiment, the fatty acid is an unbranched chain consisting of 4 - 15 carbon atoms, preferably consisting of 6 - 12 carbon atoms, or more preferably consisting of 8- 10 carbon atoms, or even more preferably consisting of 8 carbon atoms. In a most preferred embodiment, the fatty acid is caprylic acid (IUPAC name octanoic acid).

In some embodiments, the fatty acid is mixed with the organic molecule in an amount of about 0.5 - 2 mM fatty acid for 1 mg/ml of the organic molecule, such as 0.5 mM, 0.6 mM, 0.7 mM, 0.8 mM, 0.9 mM, 1 mM, 1.1 mM, 121 mM, 1.3 mM, 1.4 mM, 1.5 mM, 1.6 mM, 1.7 mM, or 2.0 mM fatty acid for 1 mg/ml. As an example, for an organic molecule solution having a concentration of 10 mg/ml, 5 - 20 mM fatty acid might be suitable. In a preferred embodiment, the fatty acid is mixed with the organic molecule in an amount of about 1 mM fatty acid for each mg/ml of the organic molecule, e.g. 10 mM fatty acid for a concentration of 10 mg/ml organic molecule solution. In a most preferred embodiment, 30 mM sodium-caprylate is mixed with a HSA solution having a concentration of 30 mg/ml.

### (b) Attaching of a targeting group

In some aspects, the nanoparticle comprises one or more targeting groups. In some aspects of the invention, the nanoparticle prepared by the inventive method comprises a targeting group, an active agent, a signaling agent, or any combination thereof.

In some embodiments, the targeting group is attached to the organic molecule before the method starts. In other embodiments, the targeting group is attached to the nanoparticle after the method of the invention has provided the nanoparticle.

The targeting group may either be non-covalently or covalently associated with the organic molecule of the nanoparticle, and can be conjugated to the organic molecule by a variety of methods.

In some embodiments, the targeting group may be either anchored in the hydrophobic portion of the organic molecule or attached to reactive terminal groups of the hydrophilic portion of the organic molecule. The targeting group may be attached to the nanoparticle via a linker to a reactive group. Preferred reactive groups include amino groups, carboxylic groups, hydrazide groups, and thiol groups. The coupling of the targeting group to the organic molecule can be performed by standard methods of organic chemistry that are known to those skilled in the art.

In certain embodiments, the total concentration of the targeting group in the final nanoparticle preparation can be from about one to about 30 targeting groups per nanoparticle.

A skilled person realizes that the systems and methods of the present invention can be employed in a variety of types of experimental, imaging, therapeutic and diagnostic procedures, including in vitro or in vivo experimental procedures. The systems and methods of the present invention can be applied to a cell or a tissue, wherein the cell can be part of a tissue, such as an inflammatory tissue or tumor tissue.

### (c) Attaching of a signaling agent

In some aspects of the invention, the nanoparticle prepared by the inventive method comprises a signaling agent. In some aspects of the invention, the nanoparticle prepared by the inventive method comprises a targeting group, an active agent, a signaling agent, or any combination thereof.

In some embodiments, the signaling agent is attached to the organic molecule before the method of preparing the nanoparticle starts. In other embodiments, the signaling agent is attached to the nanoparticle after the method of the invention has provided the nanoparticle.

### (d) Attaching of an active agent

In some aspects, the nanoparticle comprises one or more active agents. In some aspects of the invention, the nanoparticle prepared by the inventive method comprises a targeting group, an active agent, a signaling agent, or any combination thereof. In some embodiments, the active agent may be present in a nanoparticle composition.

In some embodiments, the active agent is attached to the organic molecule before the method of preparing the nanoparticle starts. In other embodiments, the targeting group is attached to the nanoparticle after the method of the invention has provided the nanoparticle.

In some embodiments, the active agent is a chemotherapy drug,antimetabolite agents(including nucleoside analogs), a cytotoxic agent (e.g. a toxin), alkylating agents, tyrosine kinase inhibitors, anthracycline antibiotics, vinca alkloids, proteasome inhibitors, taxanes, modulators of HER2/neu (such as inhibitors of HER2/neu for example Herceptin®), modulators of EGFR (such as inhibitors of EGFR for example Erbitux®), modulators of VEGFR, farnosyltransferase inhibitors, and topoisomerase inhibitors. In some embodiments, the chemotherapeutic is not a taxane(i.e., the compound is a chemotherapeutic agent other than a taxane).

The active agent described herein can be the agent itself, or a pharmaceutically acceptable salt thereof, pharmaceutically acceptable ester thereof, as well as stereoisomer, enantiomer, racemic mixture, and the like.

It is to be understood that the embodiments disclosed above in the context of the inventive nanoparticle preparation or composition *per se,* e.g. relating to specific size restrictions, surface charge, or presence and type of the targeting group, active agent or signaling agent, apply correspondingly to the present disclosure for active agent containing nanoparticles. It is only for reasons of brevity that these embodiments are not repeated here, in the context of preparation methods, the skilled person will however understand that this disclosure applies equally to these preparation methods.

An additional aspect of the invention thus also relates to a nanoparticle preparation or composition prepared by the above method.

A still additional aspect of the invention thus also relates to a nanoparticle preparation or composition preparable by the above method.

Such a composition clearly differs from, and has an advantage over, comparable nanoparticle compositions prepared by desolvation methods, i.e. not prepared by the above method, especially when such nanoparticle preparation or composition is or is rendered aqueous, as other nanoparticle preparations or compositions not prepared by the above method will typically not remain monodisperse, exhibit long-term stability, prevent aggregation, exhibit a narrow size distribution, and /or enable the uptake by the target cell. The above method thus has the important advantage of ensuring homogeneity, stability, fast cellular uptake in the resulting nanoparticle preparation or composition, even when the composition is subsequently rendered aqueous, e.g. by addition of a diluent such as water.

### (e) Measuring nanoparticle size distribution bv Photon correlation spectroscopy (PCS)

The size distribution of the nanoparticle preparation is measured by a method known as "Photon correlation spectroscopy" (alternatively also known as dynamic light scattering or quasi-elastic light scattering).PCS is a technique which can be used to determine the size-distribution profile of small particles in suspension. PCS can measure the hydrodynamic radius of enormous populations (∼10¹⁵ particles) of nanoparticles, and then models the average sizes of the particles.

In one embodiment, the nanoparticle preparation dispersed in a liquid prior to measuring the nanoparticle size using PCS. In one embodiment, the nanoparticle preparation is diluted prior to measurement using PCS. In one embodiment, the diluted nanoparticle preparation comprises up to 5% (v/v), 4% (v/v), 3% (v/v), 2% (v/v) or 1% (v/v) nanoparticles in the liquid.

In another embodiment, the nanoparticle preparation is measured using PCS before attaching a targeting moiety and/or a signaling agent to the nanoparticle.

In another embodiment, the nanoparticle measured by PCS is a stabilized nanoparticle.

In another embodiment, the nanoparticle preparation is filtered prior to PCS, wherein the filtering is performed using a 0.5 µm filter, 0.4µm filter, 0.3 µm filter, 0.2 µm filter, or 0.1µm filter.

### D. Kit

The invention also provides kits comprising the nanoparticle preparation or composition, described herein for use in the methods of treatment, methods of imaging, methods of diagnosing, and methods of detecting described herein. Kits of the invention include one or more containers comprising the nanoparticle preparation), and in some embodiments, further comprise instructions for use in accordance with any of the methods of treatment described herein. In some embodiments, the kit further comprises at least one active agent. In some embodiments, the at least one active agent comprises a chemotherapeutic agent (such as any of the chemotherapeutic agents described herein). In some embodiments, the at least one active agent comprises a hormone therapeutic agent. In some embodiments, the kit comprises i) a composition comprising a nanoparticle preparation, and ii) instructions for administering the nanoparticle preparation and the chemotherapeutic agents simultaneously and/or sequentially, for treatment of cancer, preferably carcinoma.

Instructions supplied in the kits of the invention are typically written instructions on a label or package insert (e.g., a paper sheet included in the kit), but machine-readable instructions (e.g., instructions carried on a magnetic or optical storage disk) are also acceptable. The instructions relating to the use of the nanoparticle preparation generally include information as to dosage, dosing schedule, and route of administration for the intended purpose.

The present invention also provides kits comprising nanoparticle preparations or compositions as described herein, and may further comprise instruction(s) on methods of using the nanoparticle preparation or composition, such as uses further described herein. In some embodiments, the kit of the invention comprises the packaging described above.

In other embodiments, the kit of the invention comprises the packaging described above and a second packaging comprising a buffer. It may further include other materials desirable from a commercial and user standpoint, including other buffers, diluents, filters, needles, syringes, and package inserts with instructions for performing any methods described herein.

Kits may also be provided that contain sufficient dosages of a signaling agent, targeting group, active agent, or any combination thereof.

In some embodiments, the kit comprises a dry (e.g., lyophilized) nanoparticle preparation or composition that can be reconstituted, resuspended, or rehydrated to form a stable aqueous dispersion of nanoparticles.

### E. Uses

A further aspect of the invention relates to a use of the inventive nanoparticle preparation or composition as set out herein. The use may involve formulating the inventive nanoparticle preparation or composition as set out herein in a form suitable for application to a body cavity of a subject, whose tissue is in need of monitoring, diagnosis or imaging with regards to potential tissue lesions.

In some embodiments, the body cavity is any one of colon or colorectal cavity, oral cavity, ear canal, prostate, ear-nose-throat canals, oesophagus, stomach, duodenum, jejunum, ileum, colon, sigmoid colon, rectum, anus, trachea, bronchioli and lung alveoli, vagina, cervix uteri, uterus, urethra, or urinary bladder (the space around the testis, ovary, prostate gland). In a preferred embodiment, the subject is a human.

In some embodiments, the blood space is the body cavity.

In some embodiments the body cavity is a joint cavity between bones.

The skilled person knows how to formulate a nanoparticle preparation or composition suitable for application to such a body cavity of an animal, preferably a human. In particular, the inventive nanoparticle preparation or composition may be supplied in various forms, nonlimiting examples of which comprise a powder, ointment, cream, gel, lotion, drop, spray, a dispersion, a liquid, colloidal solution, suspension, a powder, encapsulation, aerosol or gel, cream, plaster, or poultice.

To this end, the inventive nanoparticle preparation or composition may be provided as a liquid, preferably in a spray bottle, wherein the user preferably sprays a fine mist onto the surface of the body cavity, which will allow the nanoparticle preparation or composition to come into close proximity with the apical surface of the tissue to be monitored.

One aspect of the invention relates to a use of the inventive nanoparticle preparation or composition as set out herein as a tool for molecular imaging or drug delivery by applying the inventive nanoparticle preparation or composition to a body cavity of a subject. As described herein, the inventive nanoparticle preparation or composition allow the targeted delivery of the nanoparticle, which comprises an imaging agent, signaling agent and/or therapeutic/active agent, to a target site on a tissue. The inventive nanoparticle preparation or composition described herein have thus the advantage of avoiding the negative side effect associated with the nanoparticles generated by other methods, and used in other ways, predominantly by intravenous injection.

In some embodiments, the present invention is directed to methods of using any or all of the above-described nanoparticles in imaging or detecting methods, or for using an apparatus for medical imaging (e.g. radiographic instrument, X-ray, CT, PET, MRI, ultrasound).

The inventive method provided herein for producing a nanoparticle by a coacervation method, offers various advantages over nanoparticles obtained by methods known in the art, e.g. desolvation methods.

The present method is simple, and uses inexpensive starting materials, which allows a straight-forward and simple protocol for industrial upscalability, and applicability in poor countries not well equipped with highly sophisticated machinery.

Moreover, the nanoparticles obtained by the method of the invention are preferably monodisperse with a narrow size range, are very small enabling the endocytic uptake of the nanoparticles into the target cell, they are stable even under hard chemical and physical conditions (SDS gel electrophoresis, capillary electrophoresis, diafiltration), and the reaction conditions do not disturb the natural functions and conformations of the organic molecules. This renders these nanoparticles exceptionally good candidates for theranostic applications.

It is to be understood that the embodiments disclosed above in the context of the inventive nanoparticle preparation or composition *per se,* e.g. relating to specific size restrictions and surface charge of the organic nanoparticles, apply correspondingly to the present corresponding uses. It is only for reasons of brevity that these embodiments are not repeated here, in the context of uses, the skilled person will however understand that this disclosure applies equally to these uses.

### F. Nanoparticles for use in imaging methods

The methods of the present invention further include detection of lesions and/or changes in the number of lesions present in a subject, so that the disease progression may be monitored. The invention further provides that the compositions can be used to visualize, i.e., through MRI, fluorescence, fluorescence lifetime, or luminescence, the location of dysplastic tissue, inflammatory tissue, pre-cancerous or cancerous lesions in tissues of a body cavity.

In one exemplary embodiment, the nanoparticles are used during colonoscopy. Colonoscopy is carried out during screening of human populations by inserting a 1.5 meter long tube into the colon via the anus and rectum of a human body. The tube contains internal mechanisms for spraying and rinsing the colon wall and lumen, and also wire attachments for pinching off and abrading suspect lesions e.g. polyps. At the front end of a modern endoscope there is a miniature camera with appropriate illumination, for imaging the colon wall in colour. A modern colonoscope can separate colour channels with good spectral resolution and thus does not need optical filters. The surgeon inserts the endoscope into the colon until the camera reaches the Appendix vermiformis, then during the slow withdrawal of the tube he images numerous sites of the colon wall. Thus, if he sprays our nanoparticles either in large amounts into the lumen, or in small amounts directly onto the colon wall during the insertion of the endoscope, the several minutes required for uptake of the nanoparticles into the cells will pass during the final stages of insertion and the imaging can be done as the endoscope is withdrawn.

### G. Nanoparticles for use in diagnostic methods

In one aspect, the invention provides the nanoparticle preparation or composition for use in a diagnostic method practiced on the human or animal body.

In other aspects, the invention provides the nanoparticle preparation or composition for use in a method of diagnosing a disease caused by an epithelial lesion, preferably a carcinoma.

In some embodiments, the cancer is one of colorectal carcinoma, prostate cancer, hepatocellular carcinoma, breast cancer, lung cancer, small cell lung cancer, non-small cell lung cancer, or lung adenocarcinoma. In some embodiments the epithelial lesion is preferably atherosclerosis.

In one embodiment, the above diagnostic method comprises the administration of the nanoparticle preparation or composition to a body cavity of a subject. In some embodiments, the nanoparticle preparation or composition is administered directly to the body cavity of a subject by spraying the nanoparticle preparation or composition onto the surface epithelium of the body cavity. In another embodiment, the nanoparticle preparation or composition is administered directly to the body cavity of a subject, and wherein surface epithelium of the body cavity is subsequently mechanically brought into contact with the nanoparticle preparation or composition, e.g. by massage, by changing the orientation of the body, thereby allowing the nanoparticle preparation or composition to come into contact with the surface epithelium by gravity.

The present invention provides a nanoparticle preparation or composition for use in a diagnostic method in individuals to detect cancer (e.g., colorectal cancer, ovarian cancer, lung cancer, pancreatic cancer, and skin cancer (e.g., melanoma)).

### H. Use in medical methods

Various nanoparticles have been used in the art to detect or treat cancers by injecting the nanoparticles intravenously. However, any materials injected into the bloodstream are separated from the intended targets in epithelial lesions by numerous physical and chemical barriers, which form an impenetrable physical barrier lying between a capillary and an epithelium. A non-exhaustive list of the physical and/or chemical barrier comprises the endothelial cell sheet lining the blood space of the vascular system, glycosaminoglycans and glycoproteins, collagen fibrils and bundles, elastin fibers and sheets, cell bodies and cell processes, smooth muscle cell layers, and fibroblasts. These barriers effectively filter out or block passage of materials between the blood and the epithelium containing the lesion.

In addition to these barriers lying between a capillary and an target epithelium, the nanoparticle is greatly diluted when it enters the bloodstream. Only a small fraction of the nanoparticle preparation that has been injected (less than 1% of the injected bolus) has diffused across the obstacle-filled distance between the nearest capillary to the epithelial sheet containing targets, the nanoparticle is still separated from the epithelial cells by a further basement membrane. Further, if the targets are on the apical side of the epithelium - as is usually the case -the entire cell bodies of the epithelial cells, which are connected to one another by impermeable intercellular junctional apparatuses, lie between the neighboring epithelial cells. The calculated amount of the nanoparticle to reach epithelial targets is less than 0.0001%. Accordingly, the calculated efficiency of small drug molecules (∼ 2 nm diameter) reach their intended targets is approximately 0.05%, whereas the calculated efficiency of a nanoparticle of approximately 100 nm diameter, which is a relatively small nanoparticle used in the art, is far below 0.0001% and is likely to be close to zero. Importantly, most of the side-effects of any given medication are thought to be due to off-target effects, i.e. due to drug molecules circulating in the system which fail to access their intended target.

It is thus an aim of the invention to provide a nanoparticle preparation which may be administered so to circumvent the above obstacles. In particular, in view of the inefficiencies and risks accompanying injection of nanoparticles into the bloodstream, the present inventors have surprisingly found that nanoparticles of a certain size and without a surface charge may be administered directly into the body cavity, resulting in specific targeting of an epithelial lesion. Due to the application of the nanoparticle to the body cavity, off-target effects, and extreme dilution effects are minimized or eliminated. Moreover, the small size of the nanoparticles and the lack of a substantial surface charge of the nanoparticle allows the nanoparticle to be taken up by the target cell, preferably by endocytosis, which allows the detection of the site/quantity of the lesion even from outside the tissue and/or body of the subject.

The nanoparticle preparation and composition of the invention administered to a body cavity only have to cross a short barrier-free distance in order to directly access the lesion on the tissue.

In one aspect, the invention provides the nanoparticle preparation or composition for use in therapy or diagnostics.

In other aspects, the invention provides the nanoparticle preparation or composition for use in a method of diagnosing a disease caused by an epithelial lesion, preferably a carcinoma. In one embodiment, this method comprises the administration of the nanoparticle preparation or composition to a body cavity of a subject. In some embodiments, the nanoparticle preparation or composition is administered directly to the body cavity of a subject by spraying the nanoparticle preparation or composition onto the surface epithelium of the body cavity. In another embodiment, the nanoparticle preparation or composition is administered directly to the body cavity of a subject, and wherein surface epithelium of the body cavity is subsequently mechanically brought into contact with the nanoparticle preparation or composition, e.g. by massage, by changing the orientation of the body, thereby allowing the nanoparticle preparation or composition to come into contact with the surface epithelium by gravity.

In other embodiments, the invention provides the nanoparticle preparation or composition for use in a method of treating cancer, wherein said cancer is preferably carcinoma, more preferably, wherein said carcinoma is colorectal carcinoma, prostate cancer, hepatocellular carcinoma, breast cancer, lung cancer, small cell lung cancer, non-small cell lung cancer, or lung adenocarcinoma.

The present invention provides nanoparticle preparations or compositions for use in a method of treating cancer in an individual (e.g., human) comprising administering to the individual an effective amount of said nanoparticle preparation or composition comprising HSA nanoparticles. In one embodiment, the HSA nanoparticles comprise a plurality of HSA molecules, a targeting agent, a signaling agent, an active agent or any combination thereof.

Examples of cancers that may be treated by the nanoparticle preparations or compositions for use according to the invention include, but are not limited to carcinoma, more preferably, wherein said carcinoma is colorectal carcinoma, prostate cancer, hepatocellular carcinoma, breast cancer, lung cancer, small cell lung cancer, non-small cell lung cancer, or lung adenocarcinoma. In a preferred embodiment, the invention provides nanoparticle preparations or compositions for use in methods of treating colorectal cancer in an individual (e.g., human) comprising administering to the individual an effective amount of said nanoparticle preparation or composition to the colorectal body cavity.

In some embodiments, there are provided nanoparticle preparations or compositions for use in methods of treating cancer in an individual by administering to the individual (e.g., a human) an effective amount of said nanoparticle preparations or compositions, wherein the cancer is a carcinoma.

Accordingly, in some embodiments, there are provided nanoparticle preparations of compositions comprising the same for use in methods of treating cancer in an individual by administering to the individual (e.g., a human) an effective amount of said nanoparticle preparation or composition comprising nanoparticles according to the invention.

In one aspect, the invention provides a nanoparticle preparation for use in a method of treating a subject, wherein the nanoparticle preparation is administered to a body cavity of the subject. In one embodiment, the nanoparticle administered to the body cavity of the subject does not accumulate in the liver, lung, or kidney of the subject. In another embodiment, the nanoparticle administered to the body cavity of the subject is not physically or chemically altered in the liver, lung, or kidney of the subject.

In one embodiment, the invention provides a nanoparticle preparation for use in a method of detecting an epithelial target site in an organism, wherein the target site is preferably associated with a lesion, said method comprising administration of the nanoparticle preparation or composition of the invention to a body cavity; and detecting a signal emitted by the nanoparticle bound to the target site.

In one embodiment, the body cavity is selected from the group consisting of the colon or colorectal cavity, oral cavity, ear canal, prostate, ear-nose-throat canals, trachea, bronchi, bronchioli and lung alveoli, oesophagus, stomach, duodenum, jejunum, ileum, colon, sigmoid colon, rectum, anus, vagina, cervix uteri, uterus, urethra, and urinary bladder. In a preferred embodiment, the body cavity is the colon or colorectal cavity.

In some embodiments, the nanoparticle preparation for use in a method of detecting a lesion in a tissue comprises at least one target group specific for a lesion selected from the group consisting of an inflammatory lesion, a dysplastic epithelium, an early-stage carcinoma, a metastatic malignancy, an infectious lesion comprising a viral antigen, bacterial antigen, fungal antigen, protozoan antigen, or worm antigen, a pre-cancerous lesion, or a cancerous lesion, or an atherosclerotic lesion.

In some embodiments, the nanoparticle preparation for use in a method of treatment provided herein may be used to treat a subject (e.g., human) who has been diagnosed with or is suspected of having cancer. In some embodiments, the subject may be a human who exhibits one or more symptoms associated with cancer. In some embodiments, the subject may have advanced disease or a lesser extent of disease, such as low tumor burden. In some embodiments, the subject is at an early stage of a cancer. In some embodiments, the subject is at an advanced stage of cancer.

In some embodiments, the nanoparticle preparation is for use in a method of detecting a lesion during endoscopy, said method comprising the application of the nanoparticle preparation to the colorectal epithelium during endoscopy, and detecting a signal within about 30 minutes, within about 20 minutes, or within about 10 minutes.

Thus, the nanoparticle preparation or composition of the invention may be administered to a subject according to any appropriate route and regimen. In some embodiments, a route or regimen is one that has been correlated with a positive therapeutic benefit. In some embodiments, compositions described herein are administered by oral (PO), intravenous (IV), intramuscular (IM), intra-arterial, intramedullary, intrathecal, subcutaneous (SQ), intraventricular, transdermal, interdermal, intradermal, rectal(PR), vaginal, intraperitoneal (IP), intragastric (IG), topical, mucosal, intranasal, buccal, enteral, vitreal, sublingual; by intratracheal instillation, bronchial instillation, and/or inhalation; as an oral spray, nasal spray, and/or aerosol, and/or through a portal vein catheter. In a preferred embodiment, the nanoparticles are administered by non-invasive routes, e.g. orally or by inhalation. In some embodiments, the dosage form is a powder, ointment, cream, gel, lotion, drop, or spray.

In some embodiments, the exact amount administered may vary from subject to subject, depending on one or more factors as is well known in the medical arts. Such factors may include, for example, one or more of species, age, general condition of the subject, the particular composition to be administered, its mode of administration, its mode of activity, the severity of disease; the activity of the specific polysaccharide nanoparticles employed; the specific pharmaceutical composition administered; the half-life of the composition after administration; the age, body weight, general health, sex, and diet of the subject; the time of administration, route of administration, and rate of excretion of the specific compound employed; the duration of the treatment; drugs used in combination or coincidental with the specific compound employed and the like. Pharmaceutical compositions may be formulated in dosage unit form for ease of administration and uniformity of dosage. It will be understood, however, that the total daily usage of the compositions will be decided by an attending physician within the scope of sound medical judgment.

Since the nanoparticle preparation is administered to the body cavity, the nanoparticles will not come into contact with liver, kidneys and other filtration and processing mechanisms of the body, thereby no physical or chemical alteration of the nanoparticles take place. Further, the nanoparticles will not come into contact with a variety of body organs that may contain molecules cross-reactive with the target molecule, thereby minimizing the off-target side effects which are associated with the nanoparticles and methods using the same of the art. Another advantage is that the local application to a body cavity only requires dosages which are thousands of times lower as compared to intravasal applications. Further, since in some embodiments the nanoparticle comprises an imaging agent, and the uptake of the nanoparticle into the cell is in the range of minutes, the imaging and diagnostic methods are reduced to a few minutes only. All of these advantage lead to a reduced risk of toxicity associated with the nanoparticle administration.

It will be appreciated that pharmaceutical compositions described herein can be employed in combination therapies to aid in diagnosis and/or treatment. "In combination" is not intended to imply that the agents must be administered at the same time and/or formulated for delivery together, although these methods of delivery are within the scope of the embodiments described herein. Compositions can be administered concurrently with, prior to, or subsequent to, one or more other desired therapeutics or medical procedures. In will be appreciated that therapeutically active agents utilized in combination may be administered together in a single composition or administered separately in different compositions. In general, each agent will be administered at a dose and/or on a time schedule determined for that agent.

The following examples illustrate various embodiments of the invention. They are to be understood as merely illustrative, and are not to be construed as restrictive for the scope of the appended claims.

### I. EXAMPLES

The examples, which are intended to be purely exemplary of the invention and should therefore not be considered to limit the invention in any way, also describe and detail aspects and embodiments of the invention discussed above. The examples are not intended to represent that the experiments below are all or the only experiments performed. Efforts have been made to ensure accuracy with respect to numbers used (for example, amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### Example 1: Cleaning of Human Serum Albumin

Cleaning of HSA was carried out as described by Abdelmoez et al. (2010), according to Chen (1967),as follows: HSA (fraction V) was dissolved in distilled water at room temperature (50 mg/ml, pH 6.7),dry charcoal was added to the solution under continuous stirring (ratio of HSA:charcoal was 2:1 (w/w)) at pH 3. This suspension was stirred for 1 hour at4°C, filtered through a paper filter, and centrifuged at 5,000 g for 10 minutes.The supernatant was further filtered through a 0.2 µm filter and theHSA concentration was measured atUV280 nm using a spectrophotometer.

### Example 2: Preparation of sodium-caprylate

To a 10 ml solution of caprylic acid (density p = 0.91 g/ml) 12.6 ml of 5 M NaOH were added in a 1:1 ratio. The resulting precipitate was dissolved by adding 20 ml of a 150 mM NaCl solution leading to a sodium-caprylate concentration of 0.21 g/ml.

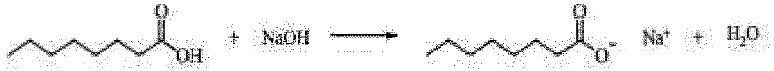

### Example 3: Stabilisation of Human Serum Albumin

Stabilisation of HSA was carried out as described by Abdelmoez et al. (2010), according to Shrake et al. (2006), as follows:
The pH of the HSA solution obtained in Example 1 was adjusted to pH 4. Then NaCl, predissolved in distilled water, was added to the solution to give a concentration of150 mM followed by addition of sodium-caprylate (ratio of HSA to sodium-caprylate = 30 mg/mIHSA to 30 mM sodium-caprylate). Then the pH was slowly adjusted to 10 by addition of 3 M NaOH. After stirring magnetically for 1 hour at room temperature at 100 rpm the pH was adjusted to 7 by addition of 0.1 M HCI and the sample was stirred overnight at room temperature.

### Example 4: Activation of protoporphyrin IX (PP IX)

5 mg PP IX diacid (0.0089 mmol) were dissolved in 1 ml DMF under light exclusion, spiked with 0.083 g EDC (1-Ethyl-3-[3-dimethyl-aminopropyl]carbodiimide hydrochloride) (50 eq, 0.433 mmol), dissolved in 0.5 ml distilled water, as well as 0.052 g NHS [(N-hydroxysulfosuccinimide (SulfoNHS), (50 eq, 0.452 mmol)], dissolved in 0.5 ml of distilled later. Activation was carried out for0.5 hours under light exclusion.

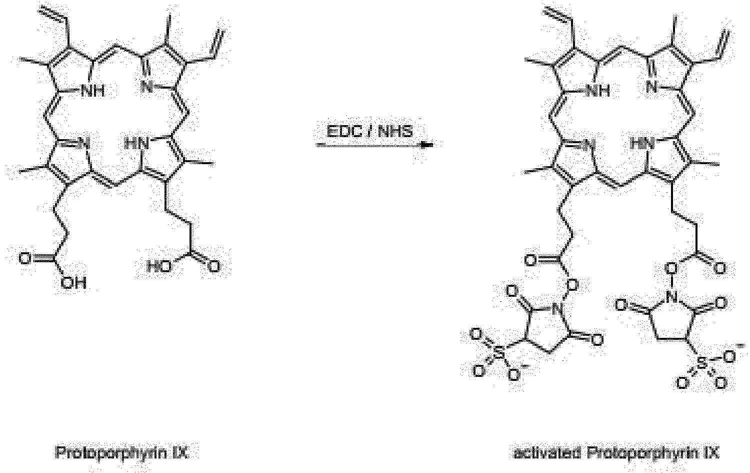

### Example 5: Attaching of activated PP IX

Two possible ways of attaching activated PP IX are possible. One method attaches the PP IX to the HSA molecules before the nanoparticle is generated (method (a) below). Alternatively, HSA nanoparticles may be produced first, which are subsequently labeled with PP IX (method (b) below). Both methods can be used interchangeably.

*Method (a): labeling HSA with PP IX before making HSA NPs:* A solution of 10 mg/ml HSA was spiked with activated PP IX diacid. The molar ratios of HSA to PP IX were between 1:4 and 1:7, the reaction was stirred overnight followed by centrifugation to remove the dark-brown precipitate that always occurs during the activation step (centrifugation conditions: step 5 for 3 minutes, step 7 for 5 minutes). The HSA-PP IX conjugate then was synthesized into nanoparticles according to the protocol of Series IV.

*Method (b) labeling HSA NP with PP IX:A* solution of HSA nanoparticles (10 mg/ml HSA) was spiked with activated PP IX diacid. The molar ratios of HSA to PP IX were between 1:4and 1:7, the reaction was stirred overnight followed by centrifugation to remove the dark brown precipitate that always occurs during the activation step (centrifugation conditions: step. 5 for 3 minutes, step 7 for 5 minutes).

### Example 6: Preparation of the nanoparticles

A solution was prepared containing either 10 mg/ml HSA or 10 mg/ml HSA-PP IX dissolved in 5 ml distilled water. Then 0.292 g of solid NaCl (1 M) were added. After complete dissolution of the salt the sample was stirred for 5 hours at 400 rpm at 50 ± 2 °C. After the sample had been stirring for 5 hours it was cooled down to room temperature (27 °C) while the stirring speed of 400 rpm was kept. As soon as the sample had reached room temperature the stirring speed was decreased to 200 rpm. The solution was subsequently spiked with 500 µl of 1 % glutaraldehyde (1:10 v/v) (Sigma) and kept stirring overnight at 200 rpm and at room temperature.

Adding 0.5 - 2 mM fatty acid, preferably 1mM fatty acid, e.g. caprylic acid, per each mg/ml of the organic molecule to stabilize the organic molecule.

The nanoparticles of the invention may be conjugated to peptides, proteins, fluorescent dye molecules, or active agents. Such conjugation is achieved by utilizing a functional group on the surface of the nanoparticle. These functional groups on the nanoparticle surface can be covalently or non-covalently conjugated to any of the above-described moieties by using standard cross-linking chemistry.

The preparation method disclosed herein comprises no phase change of the bulk solution. This is in striking contrast to desolvation coacervations used in the art.

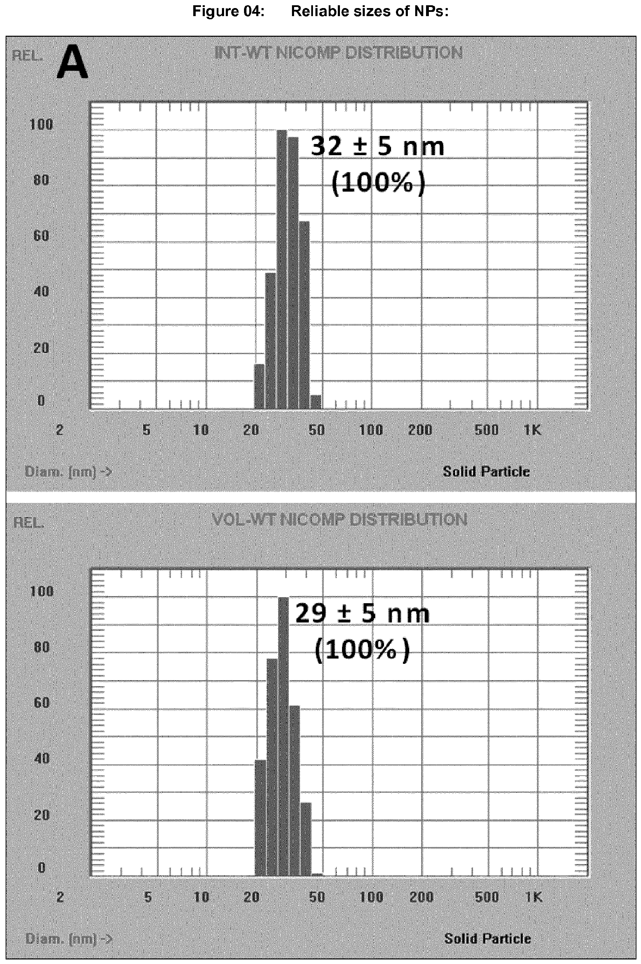

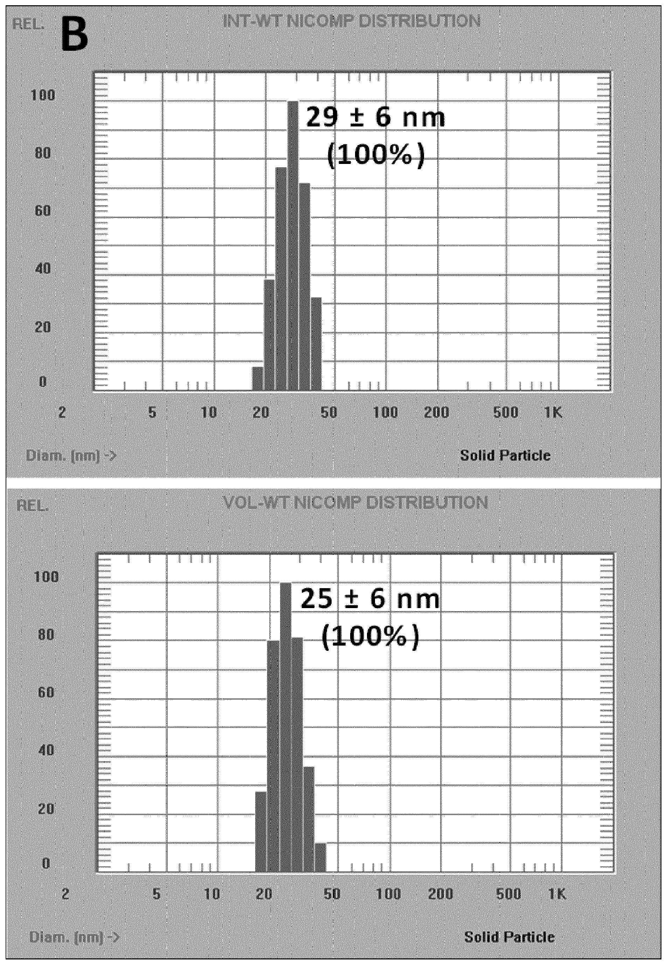

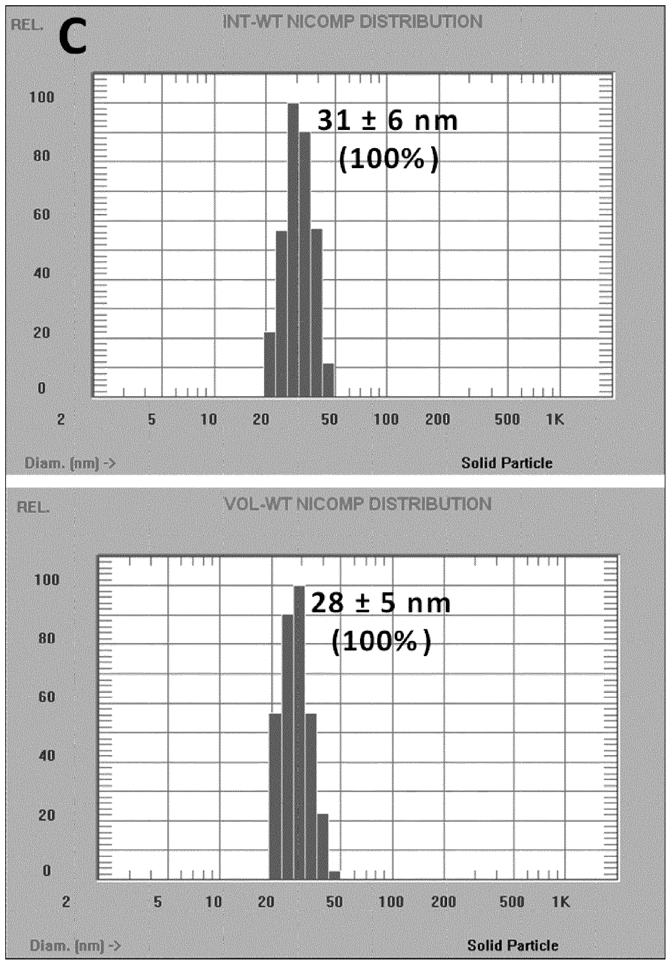

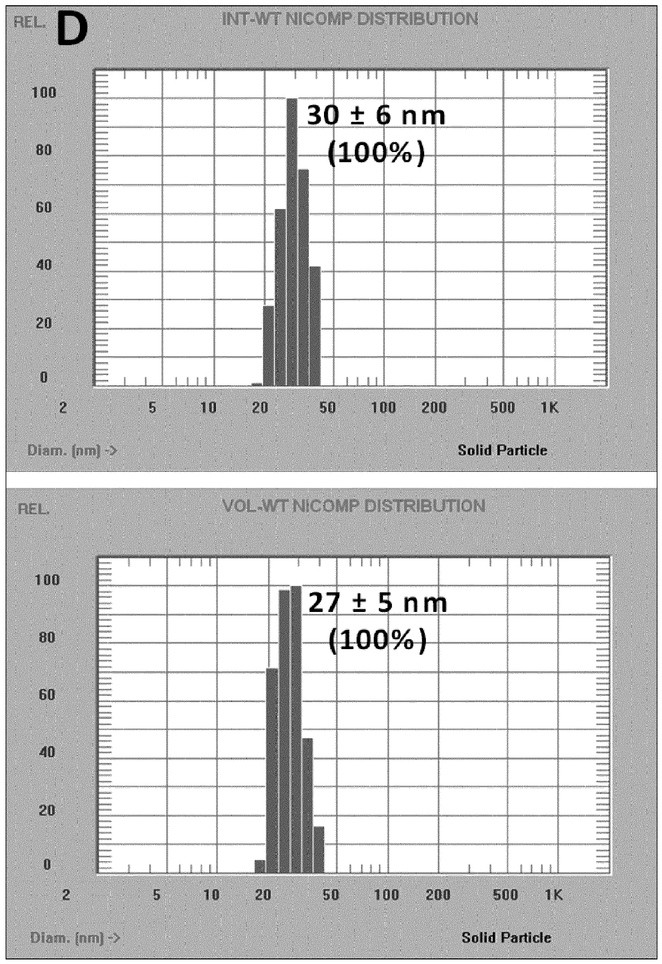

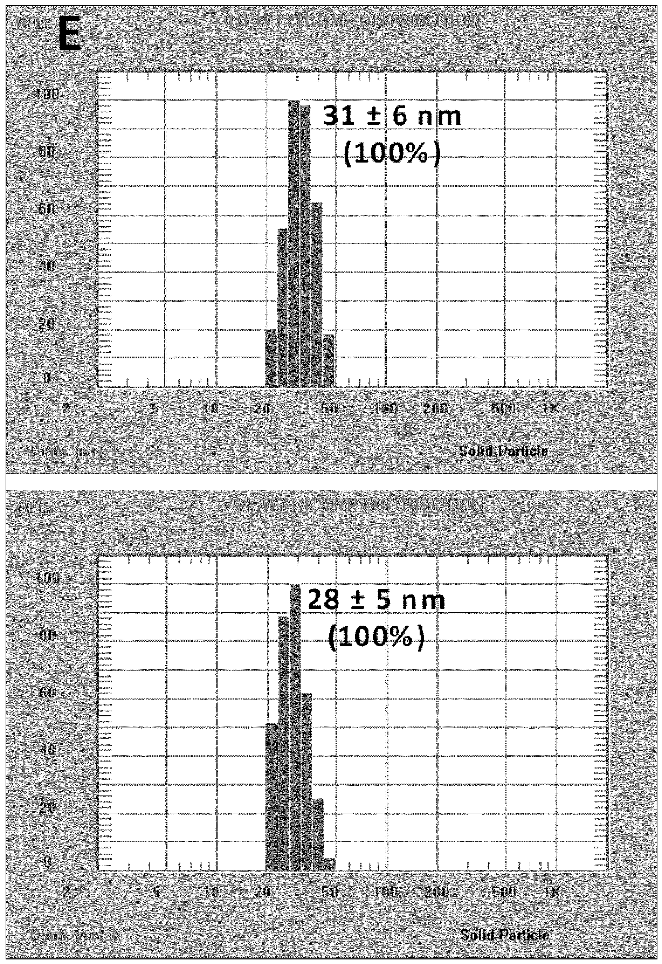

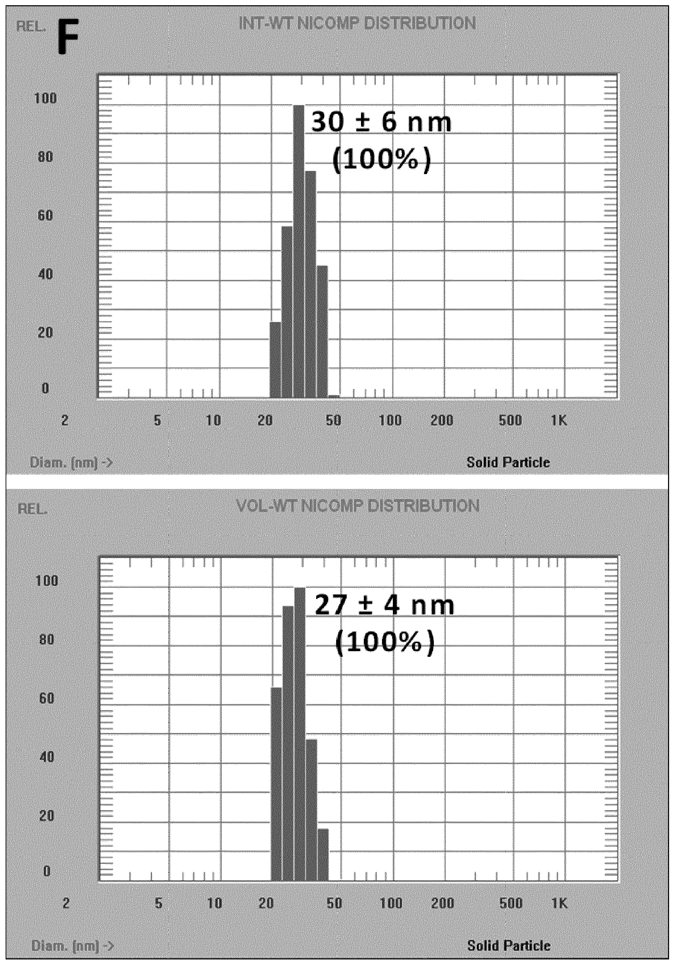

No sudden and rapid precipitation occurs during nanoparticles formation according to the invention. Spun out of a hot solution consisting only of stabilized albumin and common salt, our nanoparticles form slowly by equilibration with shear stresses and are not precipitated out in a sudden precipitation event. They are completed by a crosslinking and can then proceed immediately to further processing after simple dilution, or after diafiltration. The inventive procedure requires no repeated centrifugation and separation of supernatant from pellet. The nanoparticles produced according to the invention have strikingly narrow size distributions and this is totally reproducible across many batches (Figure 4). Other properties such as physical size and global surface charge differ strongly from the nanoparticles produced by others. The present procedure is characterized by simplicity, economy and efficiency. It only requires a weighing scale, a stirrer, a thermometer and pipettes; and can be conducted also in a general practitioner's room or a normal pharmacy.

The nanoparticles of the invention are much smaller (Figure 2) than those reported by the prior art, although Langer's group [Langer et al., 2003] invested considerable work into producing the smallest nanoparticles that were possible with their method. According to the invention; the size of the nanoparticles can be reduced to below 100 nm, preferably to about 30 nm.

Unlike the nanoparticles known in the art, the inventive nanoparticles bear no surface charge. In contrast, the nanoparticles generated by other methods known in the art reported typically have a negative charge of about -40 mV.

The nanoparticles of the invention are stable at pH values between 4 and 8, whereas the nanoparticles produced by the desolvation method routinely used in the art (according to Langer [Langer et al., 2003] are unstable above pH 7.

In addition, the nanoparticles of the invention are extremely stable, allowing storing the nanoparticle preparation for longer than 2 years at 4°C without detectable deterioration. In contrast, the nanoparticles of the art are only stable for a short period of time (up to 2 months; Galisteo-González & Molina-Bolivar).

### Example 7: Targeting of fluorescent HSA and of fluorescent HSA nanoparticles

### 1. Targeting of HSA / covalently attached PP IX with anti-EpCAM antibody:

The pH of the sample containing HSA with covalently attached PP IX was adjusted to 6.5 with 0.1 M HCI. The original concentration of HSA for labelling with PP IX was 10 mg/ml HSA, after purification (centrifugation) the concentration was about 3.4 mg/ml HSA which was taken for further calculation for attaching the linker and the antibody. 100 µl of said HSA / PP IX were taken and diluted with 400 µl distilled water (to have some volume for proper stirring). Then a stock solution of 1 mg/ml KMUH ((N-[κ-maleimidoundecanoic acid] hydrazide, trifluoroacetic acid salt), molecular weight 409.40 g/mol, spacer arm 19.0 Å) in DMF was prepared of which 16.2 µl were taken and added to the HSA / PP IX solution. The sample containing HSA / PP IX and KMUH was stirred for 2 hours at room temperature under light exclusion.

45 minutes before the above described reaction was completed the carbohydrate chain of the anti-EpCAM antibody was activated with sodium meta-periodate. 2.5 ml of anti-EpCAM antibody (2.125 mg, 3.036 x 10⁻⁸ mol) were cooled to about 4 °C in an ice-water bath under continuous stirring. Then a stock solution of 1 mg/ml sodium meta-periodate was prepared in distilled water; 188 µl were taken out (188 µg, 8.79 x 10-7 mol) and added to the antibody. The sample was stirred vigorously for 30 minutes at 4 °C under light exclusion and then was added to the HSA / PP IX / KMUH sample. This mixture was then stirred for 2 hours at room temperature under light exclusion before testing the antibody efficiency on placenta sections on glass slides (see below).

### 2. Targeting of HSA / covalently attached PP IX nanoparticles with anti-EpCAM antibody:

1 ml of HSA-PP IX nanoparticles (0.01 g HSA, 0.0015 mmol) was diluted with 4 ml distilled water (0.01 g in 5 ml; 0.002 g/ml). The pH was adjusted to 6.5 with 0.1 M NaOH. Then a stock solution of 1 mg/ml KMUH ((N-[κ-maleimidoundecanoic acid] hydrazide, trifluoroacetic acid salt), molecular weight 409.40 g/mol, spacer arm 19.0 Å) in DMF was prepared of which 430 µl were taken and added to the HSA / PP IX nanoparticle solution(HSA : KMUH = 1 : 7). The sample containing HSA / PP IX nanoparticles and KMUH was stirred for 2 hours at room temperature under light exclusion.

45 minutes before the above described reaction was completed the carbohydrate chain of the anti-EpCAM antibody was activated with sodium meta-periodate. 4.875 ml of anti-EpCAM antibody (8.885 mg, 5.92 x 10-8 mol; this number reflects the true molecular weight of anti-EpCAM antibody, it was recalculated with 150,000 g instead of 70,000 g for antibody weights) were cooled to ∼ 4 °C in an ice-water bath under continuous stirring. Then a stock solution of1.8 mg sodium meta-periodate was prepared in 5 ml distilled water and cooled in an ice-bath;75 µl were taken out (27 µg, 1.27 x 10-7 mol, anti-EpCAM antibody : sodium metaperiodate = 1 : 2,1; the relation of anti-EpCAM antibody : sodium meta-periodate should be1 : 1, but as the antibody weight unfortunately was calculated with 70,000 g instead of150,000 g the relation, now being recalculated using the correct numbers, is 1 : 2.1) and added to the antibody. The sample was stirred vigorously for 30 minutes at 4 °C under light exclusion and then was added to the HSA / PP IX / KMUH sample (HSA : anti-EpCAM antibody = 1 : 7).

### Example 8: Measurement of nanoparticle size

The nanoparticle of the invention is characterized i.a. by its uniform and small size. The size of the nanoparticle may be measured by various methods known in the art.

One reliable method is Photon Correlation Spectroscopy (PCS), which measures the hydrodynamic radius of nanoparticles and microparticles. PCS is advantageous because it is able to routinely measure enormous populations (∼10¹⁵ particles) in a timely manner, and then models the average sizes of the particles.

### Results

### HSA plus activated PP IX

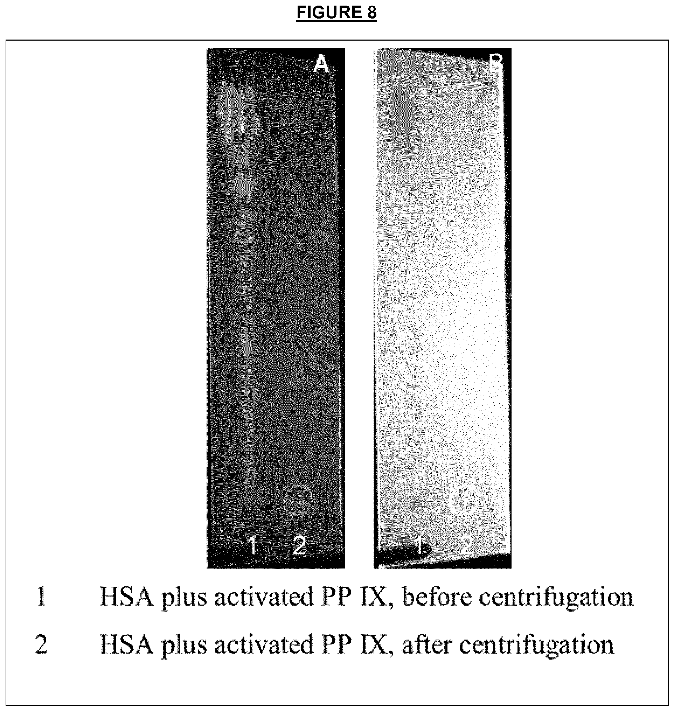

100 mg Human Serum Albumin (0.0015 mmol) were dissolved in 8 ml distilled water. Activated PP IX (0.0034 g or 2.267 ml, 0.006 mmol) was added dropwise and the sample was stirred at 200 rpm at room temperature over night. The sample was centrifuged the next day (step 5 for 3 minutes), the supernate was taken and centrifuged again (step 7 for 5 minutes). Total sample volume after centrifugation was 9.5 ml. Afterwards a TLC was conducted using a mixture of CHCl3 and MeOH in a 9:1 ratio plus 85.5 µl of acetic acid as running solvent. The images below show the dots before development (**Figure 8**), and the results after development at wavelength 366 nm (**Figure 8**).

Centrifugation removed unbound PP IX impurities, the bound PP IX is stably connected with HSA.

### HSA plus activated PP IX formed into nanoparticles

PCS-Data of coacervated HSA nanoparticles carrying PP IX: intensity was at about 1100 kHz and showed good stability, dilution necessary, stability remained; Fit Error 45.74, Residual 53.86, Chi Squared 8.43, Min. Diameter 2, Plot Range 1000, threshold 0 %.

To evaluate the fluorescence intensities the sample was applied to different wavelengths, 382 nm,404 nm and 490 nm respectively. The highest intensity was seen at A 404 nm.

Comparison of HSA-PP IX nanoparticles with Nile Red PACA and PP IX PACA nanoparticles from Trondheim: the applied wavelength onto the samples was 404 nm.

### SDS gel electrophoresis with HSA/PP IX (1:4) nanoparticles using different linkers

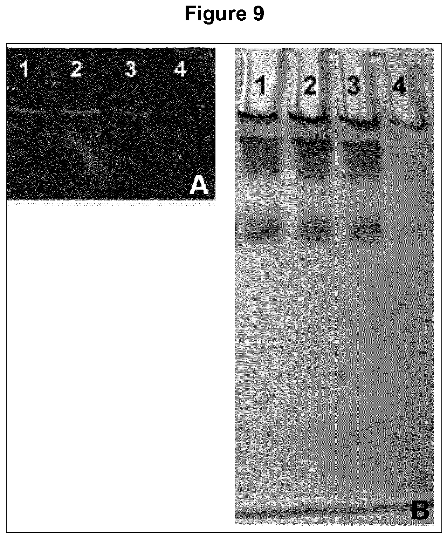

Different linking molecules analogue to glutaraldehyde were tested and compared with glutaraldehyde:
An SDS-polyacrylamide gel electrophoresis using a self-casted gel was conducted using 50 V for10 minutes, 120 V for 70 minutes, UV light at λ366 nm, Coomassie Blue staining for 1 hour, destaining for 1 hour (each sample: 8 µl, plus 32 µl H2O plus 10 µl sample buffer; 95 °C for 5 minutes; 20 µl of each sample were loaded into the respective well) **Figure 9****.**

Activated PP IX is stably connected to HSA, nanoparticle synthesis did not disturb this connection. The best linker for nanoparticle formation seems to be glutaraldehyde **(****Figure 9****),**

### Covalent attachment of anti-EpCAM antibody to HSA/PP IX nanoparticles

To attach anti-EpCAM antibody to the nanoparticles covalently a linker was used. The concept was to use the one sulfhydryl-group that is present on each HSA molecule to assure a defined binding of antibodies on the nanoparticles. The linker should not disturb the binding sites of the antibody, so it should react with the carbohydrate chain of the antibody and not with carboxyl or amino-groups. A literature search found a linker commercially available from ThermoFischer, i.e. KMUH:

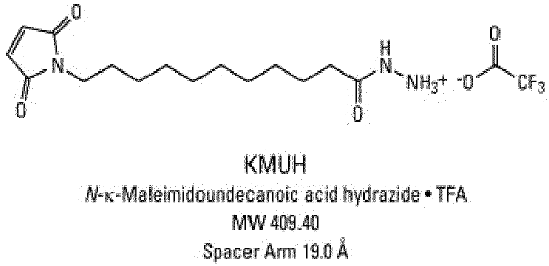

The maleimide group attaches covalently to the SH-group of HSA, the hydrazide attaches covalently to the carbohydrate of the antibody (see image below).

### Specific targeting of nanoparticle to target site

Anti-EpCAM antibody was covalently attached to HSA/PP IX and to HSA/PP IX nanoparticles, and tested on human tissue sections.

### Fluorescence imaging

Colon carcinoma tissues in the form of histopathological sections were prepared by dewaxing and rehydration and then incubated with the nanoparticles as described in Methods section. In a light microscope equipped with a non-standard fluorescence optical arrangement bright patches of pink fluorescence were present at many sites on the tissue section, whereas there were also large areas of the section in which no fluorescence was observable.

EpCAM-targeted nanoparticles, and that the nanoparticles were binding in a distribution that was clearly specific, and indeed specific in a pattern expected for EpCAM distribution in a colon carcinoma. Already the absence of fluorescent labelling across large areas of the section indicated that this labelling was specific, and the pattern of labelling was consistent with that expected for EpCAM distribution in a section of highly malignant colon cancer tissue.

An analysis of fluorescent labelling of colon carcinoma tumour cells by PP9-bearing EpCAM-targeted nanoparticles is shown in **Figure 10****.**

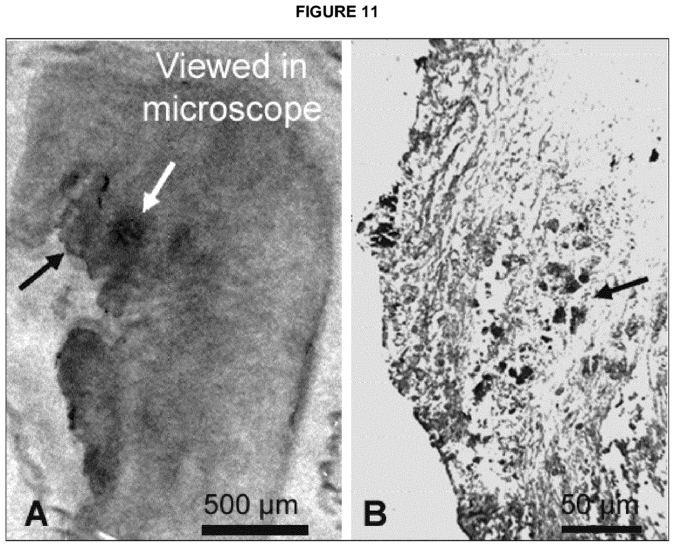

A third step was carried out. The slide examined for PP9 fluorescence was fixed briefly in low-dilution glutaraldehyde, the aldehyde was quenched, and the slide then incubated with an antibody specific for HSA. A subsequent immunoperoxidase incubation to reveal the primary anti-HSA antibody was carried out, and the slide was then incubated in a chromogenic diaminobenzidene/hydrogen peroxide solution. In a fourth step the slide was dehydrated through xylols and embedded in a usual mounting medium (DePeX) under a coverslip. After 3 days it was viewed at higher power in a light microscope, the brightly pink (fluorescent) regions and the brown (immunoperoxidase) regions of the section being found to contain labelled (tumour) cells, whereas most of the tissue was weakly labelled or not labelled **(****Figure 11****).** This distribution of label matches the expected distribution of EpCAM, which is expressed in the early-malignant cells but not in the post-EMT cells in the section. This data linked the fluorescence data visible in a commercial camera (inferior to a modern endoscope) with nanoparticle labelling of the tumour cells in the tissue.

Control experiments of the absorption type were not carried out. Instead, a series of other steps were carried out to demonstrate specific labelling by the targeted nanoparticles.

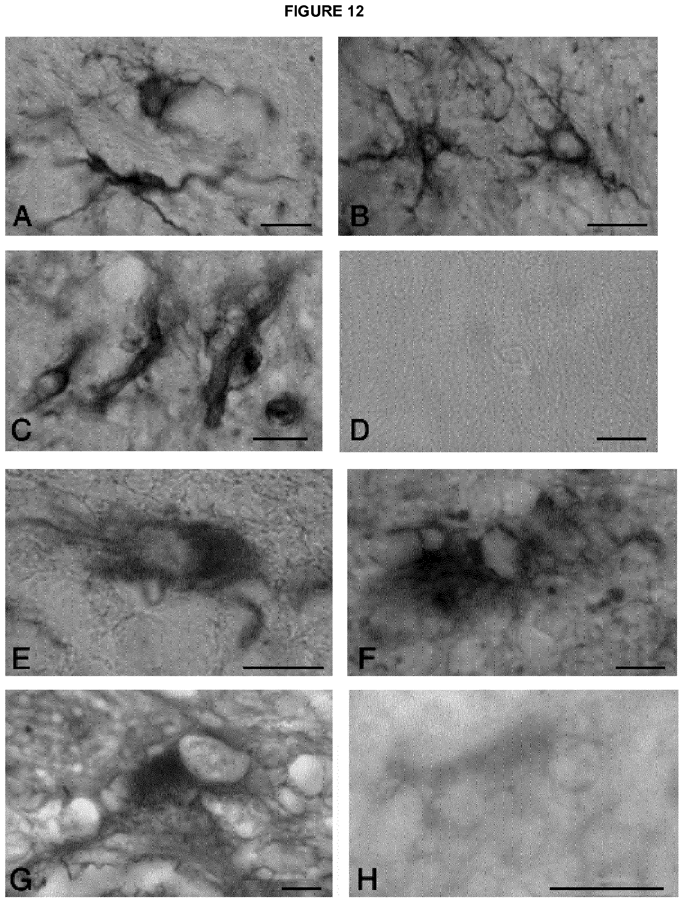

A fifth step was made by use of anti-GFAP (**Figure 12**).

### Control experiments for targeting specificity

PP-IX-bearing HSA nanoparticles received attachments of two different targeting groups: an anti-EpCAM mouse monoclonal antibody and an anti-GFAP rabbit polyclonal antibody. The two differently targeted nanoparticle solutions were incubated in cross-incubation protocols with brain tissues or with brain cancer tissues, to test for binding to GFAP which is a characteristic molecule of astroglial. They were also incubated with various EpCAM-containing tissues, such as colon carcinoma or kidney tissues. The results matched expectations, showing that the GFAP-targeted nanoparticles labelled astroglial in the brain and glioma tissues but labelled nothing in the kidney, whereas the EpCAM-targeted nanoparticles bound to tubuli in the kidney tissues and to tumour cells in colon carcinoma tissues, but labelled nothing in the brain tissues or the glioma tissues. Details of the incubations are provided here:
The target molecule was Glial Fibrillary Acidic Protein (GFAP), which forms the intracellular intermediate filaments within astrocytes in the central nervous system. It is a highly conserved protein, and has been used to track astroglial cells in numerous mammalian species including human. It is present only within the intermediate filaments (10-12 nm diameter) within the cells, the remainder of the cytoplasm does not label for this protein. A typical immunoperoxidase image is shown in **12A**, and was produced in a 2-step labelling procedure using a commercially available rabbit polyclonal antibody specific for GFAP as first antibody incubation and a commercially available anti-rabbit antibody-peroxidase conjugate as second antibody incubation. The procedures used to obtain images **12A-12D** are tabulated here:

| **Tissue, a dewaxed microtome section of *Callithrix* brain** | **First "antibody" incubation** | **Second antibody incubation** | **Third antibody incubation** | **Chromogenic development in DAB/H₂O₂ and mounted in DePex** | **Microscope objective used; Calibration bar** | **What is visualized in the image** |
|---|---|---|---|---|---|---|
| **Image A** | Rb-αGFAP | Gt-αRb-peroxidase | None | Yes | x63 oil | GFAP intermediate filaments in the astroglial cells |
| | | | | | 10 µm | |
| **Image B** | Rb-α GFAP-targeted PP9-bearing HSA nanoparticles | Gt-αRb-peroxidase | None | Yes | x63 oil | The targeting group attached to the nanoparticles |
| | | | | | 10 µm | |
| **Image C** | Rb-α GFAP-targeted PP9-bearing HSA nanoparticles | Ms-anti-HSA | Rb-αMs-peroxidase | Yes | x40 oil | The HSA comprising the nanoparticles a |
| | | | | | 10 µm | |
| **Image D** | None | Ms-anti-HSA | Rb-αMs-peroxidase | Yes | x40 oil | Negative control for nanoparticle HSA |
| | | | | | 10 µm | |

In this Table all abbreviations appear in a form easily recognized by a person skilled in the art.

The data shown in Figure 16 and detailed in this Table show that the images obtained by use of GFAP-targeted HSA nanoparticles (bearing PP9) are identical with those obtained by use of a commercial GFAP-targeted antibody, and this is true whether the HSA body of the nanoparticle is targeted in this immunohistochemistry, or whether the targeting group attached to that HSA body is targeted. Omission of the nanoparticles from the protocol results in lack of labelling, this is known as the "primary antibody" negative control.

The images **12E** - **12H** show sections of a glioma tumour removed from a human patient. The GFAP protein is expressed in the characteristic cells of gliomas, that is, in malignant astroglial cells. Since these cells are more extensively spread spatially than are the astroglial cells in normal brain tissues, it is often possible to discern the individual GFAP intermediate filaments in labelled glioma tissues. The procedures used to obtain images **12A-12D** are tabulated here:

| **Tissue, a dewaxed microtome section of a human glioma** | **First "antibody" incubation** | **Second antibody incubation** | **Third antibody incubation** | **Chromogenic development in DAB/H₂O₂ and mounted in DePex** | **Microscope objective used; Calibration bar** | **What is visualized in the image** |
|---|---|---|---|---|---|---|
| **Image E** | Rb-αGFAP | Gt-αRb-peroxidase | None | Yes | x40 oil | GFAP intermediate filaments in the astroglial cells |
| | | | | | 10 µm | |
| **Image F** | Rb-α GFAP-targeted PP9-bearing HSA nanoparticles | Gt-αRb-peroxidase | None | Yes | x40 oil | The targeting group attached to the nanoparticles |
| | | | | | 10 µm | |
| **Image G** | Rb-α GFAP-targeted PP9-bearing HSA nanoparticles | Ms-anti-HSA | Rb-αMs-peroxidase | Yes | x40 oil | The HSA comprising the nanoparticles a |
| | | | | | 10 µm | |
| **Image H** | None | Ms-anti-HSA | Rb-αMs-peroxidase | Yes | x40 oil | Negative control for nanoparticle HSA |
| | | | | | 10 µm | |

### REFERENCES

Langer K, Balthasar S, Vogel V, Dinauer N, Von Briesen H, Schubert D (2003) Optimization of the preparation process for human serum albumin (HSA) nanoparticles. International Journal of Pharmaceutics 257: 169-180
Ogura E, Senzaki H, Yoshizawa K, Hioki K, Tsubura A (1998) Immunohistochemical localization of epithelial glycoprotein EGP-2 and carcinoembryonic antigen in normal colonic mucosa and colorectal tumors. Anticancer Res 18: 3669-3675
Peters T Jr (1996) All about albumin. Biochemistry, Genetics, and Medical Applications. Academic Press, London. 455 pp.
Debbage P & Thurner (2012) Transbarrier targeting in the intestine: nanomedical options in oncology. Int J Clin Pharmacol & Ther/2012: 55-64
Thomasin C, Nam-Trân H, Merkle HP, Gander B (1998) Drug microencapsulation by PLA/PLGA coacervation in the light of thermodynamics. 1. Overview and theoretical considerations. J of Pharmaceutical Sciences 87: 259 - 268
Thurner GC (2014) Development of albumin-based nanoparticles for molecular imaging and therapy of diseases: a multidisciplinary challenge in integrating and applyingchemistry, physics and mathematics at nano-, micro- and macro-scales in biology. Dissertation for Doctor of Philosophy degree at the Medical University of Innsbruck, Austria, July 2014.
Thurner GC, Debbage P (2018) Molecular imaging with nanoparticles: the dwarf actors revisited 10 years later. Histochem Cell Biol 150: 733 - 794
Trzpis M, McLaughlin PMJ, de Leij LMFH, Harmsen MC (2007) Epithelial Cell Adhesion Molecule. More than a carcinoma marker and adhesion molecule. Am J Pathol 171: 386-395
Xie X, Wang CY, Cao YX, Wang W, Zhang R, Chen LH, Dang NN, Fang L, Jin BQ. Expression pattern of epithelial cell adhesion molecule on normal and malignant colon tissues. World J Gastroenterol 2005; 11(3): 344-347 http://www.wjgnet.com/1007-9327/11/344.asp

## Claims

1. A nanoparticle preparation for molecular imaging or drug delivery, comprising organic nanoparticles having a diameter of less than about 100 nm, preferably about 10 -about 80 nm, more preferably about 20 - about 60 nm, even more preferably about 20 - about 50 nm, most preferably about 30nm,
wherein at least 90% of the organic nanoparticles have a diameter within 25 nm of the average diameter of the organic nanoparticles comprised in the nanoparticle preparation,
wherein the nanoparticle preparation is substantially monodisperse, and
wherein the surface charge of the organic nanoparticles at pH 7 is about zero.

2. The nanoparticle preparation according to claim 1, wherein the organic nanoparticle is a protein nanoparticle, preferably wherein the protein nanoparticle is a human serum albumin (HSA) nanoparticle, insulin nanoparticle, or a nanoparticle made from a non-glycosylated protein.

3. The nanoparticle preparation according to any of the preceding claims, wherein the organic nanoparticle comprises a signaling agent, wherein the signaling agent is preferably selected from the group consisting of photoluminescence agents, ultrasound, microwave and radio-wavelength emitters, radionuclides, fluorescent dyes, chemiluminescent agents, bioluminescent agents, spectrally resolvable inorganic fluorescent semiconductors nanocrystals and quantum dots.

4. The nanoparticle preparation according to any of the preceding claims, wherein the organic nanoparticle comprises a targeting group specific for a target site within a subject, preferably wherein the targeting group is an antibody or fragment thereof, a peptide, an oligonucleotide, an aptamer, a nucleotide, or a lectin.

5. The nanoparticle preparation according to claim 4, wherein the targeting group is specific for a target site on an inflammatory lesion, a tumor antigen, a dysplastic epithelium, a metastatic malignancy, an infectious antigen such as a viral antigen, bacterial antigen, fungal antigen, protozoan antigen, or worm antigen, a pre-cancerous lesion, or a cancerous lesion.

6. The nanoparticle preparation according to any one of the preceding claims, further comprising an active agent attached to the organic nanoparticle.

7. A composition comprising the nanoparticle preparation according to any one of the preceding claims, wherein said composition is in form of a dispersion, a liquid, colloidal solution, suspension, a powder, encapsulation, aerosol or gel, cream, plaster, ointment, lotion, drop, spray or poultice.

8. The nanoparticle preparation or composition according to any one of the preceding claims for use in therapy or diagnostics.

9. The nanoparticle preparation according to any one of claims 1 - 6, or the composition according to claim 7, for use in a method of diagnosing a disease associated with an epithelial lesion, wherein said disease is preferably an atherosclerotic plaque, or a carcinoma.

10. The nanoparticle preparation according to any one of claims 1 - 6, or the composition according to claim 7, for use in a method of treating cancer, wherein said cancer is preferably carcinoma, more preferably, wherein said carcinoma is colorectal carcinoma, prostate cancer, hepatocellular carcinoma, breast cancer, lung cancer, small cell lung cancer, non-small cell lung cancer, lung adenocarcinoma, bladder cancer, melanoma, ovarian cancer, gastric cancer, cancer of the upper ENT tracts, cervical cancers, ovarian cancer, uterine/endometrial cancer, vaginal cancer, vulvar cancer, gestational trophoblastic disease (GTD), primary peritoneal cancer, or ocular tumors, such as eye melanoma, retinal pigment epithelium (RPE) tumors, retinoblastoma.

11. A method of making a nanoparticle preparation, said method comprising the steps of
a) Providing an aqueous solution of an organic molecule in its native conformation at pH 4 to pH 8, and at a concentration of about 1 mg/ml to about 50 mg/ml, wherein the organic molecule is preferably a protein, more preferably HSA;
b) Adding an inorganic salt, preferably an alkali salt such as sodium chloride, to a final concentration of 0.5 to 2 M under continuous fast stirring and at a temperature of about 40 to about 65 °C;
c) Cooling the solution to ambient temperature; and
d) Adding a cross-linking agent at a final concentration of about 0.05 %(w/v) to 0.2 % (w/v), preferably wherein the final concentration is 0.1 % (w/v), and further wherein the cross-linking agent is preferably glutaraldehyde, 1,5-dichloropentane or glutaryl chloride.

12. A nanoparticle obtainable by the method of claim 11.

13. A method of detecting an epithelial target site in an organism, wherein the target site is preferably associated with a lesion, said method comprising
• administration of the nanoparticle preparation or composition according to any one of claims 1 - 7 to a body cavity; and
• detecting a signal emitted by the nanoparticle located at the target site,
wherein the lesion is preferably selected from the group consisting of an inflammatory lesion, a dysplastic epithelium, an early-stage carcinoma, a metastatic malignancy, an infectious lesion comprising a viral antigen, bacterial antigen, fungal antigen, protozoan antigen, an atherosclerotic lesion, a pre-cancerous lesion, and a cancerous lesion.

14. A method for imaging an epithelial lesion, said method comprising
• administration of the nanoparticle preparation or composition according to any one of claims 1 - 7 to a body cavity; and
• detecting a signal emitted by the nanoparticle located at the target site of an epithelial lesion;
wherein the body cavity is preferably selected from the group consisting of the colon, ear canal, prostate, oral cavity, ear-nose-throat canals, oesophagus, stomach, duodenum, jejunum, ileum, colon, sigmoid colon, rectum, anus, trachea, bronchioli and lung alveoli, vagina, cervix uteri, uterus, urethra, urinary bladder, testis in peritoneal sac and joint cavities and outer, anterior and posterior ocular spaces/cavities, spinal and central nervous system (CNS) cavities, and the surface of the skin;
wherein the preferred mode of administration of the nanoparticle preparation to the body cavity occurs orally, intravenously, intramuscularly, intraperitoneally, subcutaneously, by inhalation, intra-arterially, intramedullary, intrathecally, subcutaneously, transdermally, interdermally, intradermally, rectally, vaginally, intraperitoneally, intragastrically, topically, mucosally, intranasally, buccally, enterally, vitreally, sublingually, by intratracheal instillation, bronchial instillation, and/or inhalation; as an oral spray, nasal spray, and/or aerosol, and/or through a portal vein catheter.

15. Use of a nanoparticle preparation or composition according to any one of claims 1 - 7 for imaging or detecting a lesion.


SEQUENCE LISTING

<110> Stams Diagnostics - Debbage, Kayatz & Thurner GesbR

<120> Suppressed for reasons of confidentiality

<130> EP124356-FZ298

<160> 2

<170> PatentIn 3.5

<210> 1
<211> 609
<212> PRT
<213> Homo sapiens

<220>
<223> P02768 HUMAN Serum albumin Isoform 1

<400> 1


<210> 2
<211> 417
<212> PRT
<213> Homo sapiens

<220>
<223> P02768 HUMAN Serum albumin Isoform 2

<400> 2
Met Lys Trp Val Thr Phe Ile Ser Leu Leu Phe Leu Phe Ser Ser Ala
1               5                   10                  15
Tyr Ser Arg Gly Val Phe Arg Arg Asp Ala His Lys Ser Glu Val Ala
            20                  25                  30
His Arg Phe Lys Asp Leu Gly Glu Glu Asn Phe Lys Ala Trp Ala Val
        35                  40                  45
Ala Arg Leu Ser Gln Arg Phe Pro Lys Ala Glu Phe Ala Glu Val Ser
    50                  55                  60
Lys Leu Val Thr Asp Leu Thr Lys Val His Thr Glu Cys Cys His Gly
65                  70                  75                  80
Asp Leu Leu Glu Cys Ala Asp Asp Arg Ala Asp Leu Ala Lys Tyr Ile
                85                  90                  95
Cys Glu Asn Gln Asp Ser Ile Ser Ser Lys Leu Lys Glu Cys Cys Glu
            100                 105                 110
Lys Pro Leu Leu Glu Lys Ser His Cys Ile Ala Glu Val Glu Asn Asp
        115                 120                 125
Glu Met Pro Ala Asp Leu Pro Ser Leu Ala Ala Asp Phe Val Glu Ser

130                 135                 140
Lys Asp Val Cys Lys Asn Tyr Ala Glu Ala Lys Asp Val Phe Leu Gly
145                 150                 155                 160
Met Phe Leu Tyr Glu Tyr Ala Arg Arg His Pro Asp Tyr Ser Val Val
                165                 170                 175
Leu Leu Leu Arg Leu Ala Lys Thr Tyr Glu Thr Thr Leu Glu Lys Cys
            180                 185                 190
Cys Ala Ala Ala Asp Pro His Glu Cys Tyr Ala Lys Val Phe Asp Glu
        195                 200                 205
Phe Lys Pro Leu Val Glu Glu Pro Gln Asn Leu Ile Lys Gln Asn Cys
    210                 215                 220
Glu Leu Phe Glu Gln Leu Gly Glu Tyr Lys Phe Gln Asn Ala Leu Leu
225                 230                 235                 240
Val Arg Tyr Thr Lys Lys Val Pro Gln Val Ser Thr Pro Thr Leu Val
                245                 250                 255
Glu Val Ser Arg Asn Leu Gly Lys Val Gly Ser Lys Cys Cys Lys His
            260                 265                 270
Pro Glu Ala Lys Arg Met Pro Cys Ala Glu Asp Tyr Leu Ser Val Val
        275                 280                 285
Leu Asn Gln Leu Cys Val Leu His Glu Lys Thr Pro Val Ser Asp Arg
    290                 295                 300
Val Thr Lys Cys Cys Thr Glu Ser Leu Val Asn Arg Arg Pro Cys Phe
305                 310                 315                 320
Ser Ala Leu Glu Val Asp Glu Thr Tyr Val Pro Lys Glu Phe Asn Ala
                325                 330                 335
Glu Thr Phe Thr Phe His Ala Asp Ile Cys Thr Leu Ser Glu Lys Glu
            340                 345                 350
Arg Gln Ile Lys Lys Gln Thr Ala Leu Val Glu Leu Val Lys His Lys
        355                 360                 365
Pro Lys Ala Thr Lys Glu Gln Leu Lys Ala Val Met Asp Asp Phe Ala
    370                 375                 380
Ala Phe Val Glu Lys Cys Cys Lys Ala Asp Asp Lys Glu Thr Cys Phe
385                 390                 395                 400
Ala Glu Glu Gly Lys Lys Leu Val Ala Ala Ser Gln Ala Ala Leu Gly
                405                 410                 415
Leu